(19) Europäisches Patentamt
European Patent Office
Office européen des brevets

(11) **EP 2 431 739 A1**

(12) **EUROPEAN PATENT APPLICATION**

(43) Date of publication:
**21.03.2012 Bulletin 2012/12**

(51) Int Cl.:
*G01N 33/49* (2006.01)　　　*G01N 33/86* (2006.01)

(21) Application number: **10306008.3**

(22) Date of filing: **21.09.2010**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO SE SI SK SM TR**<br>Designated Extension States:<br>**BA ME RS**<br><br>(71) Applicant: **SANOFI**<br>**75013 Paris (FR)** | (72) Inventor: **The designation of the inventor has not yet been filed**<br><br>(74) Representative: **Körner, Kathrin et al**<br>**Sanofi-Aventis Deutschland GmbH**<br>**Patente Deutschland**<br>**Industriepark Höchst**<br>**Gebäude K801**<br>**65926 Frankfurt am Main (DE)** |

(54) **Method for the detection and/or quantification of the interaction of platelets with interaction partners**

(57)　The invention refers to methods for detecting and/or quantifying the interaction of platelets or platelet surface molecules with an interaction partner, methods for detecting and/or quantifying the capability of a test substance to modulate the interaction of platelets or platelet surface molecules with interaction partners and articles of manufacture.

Fig. 3

not activated　　　activated (filopodia!)

HC well　　　　LC well　　　　vWF-A1 + OS-1

EP 2 431 739 A1

**Description**

Method for the detection and/or quantification of the interaction of platelets with interaction partners

Present invention relates to a method for the detection and/or quantification of the interaction of platelets or platelet surface molecules with interaction partners, a method for the detection and/or quantification of the capability of a test substance to modulate the interaction of platelets or platelet surface molecules with interaction partners and to an article of manufacture.

The most common cause of death in first world countries is acute myocardial infarction as caused by coronary artery thrombosis. Platelet adhesion and thrombus formation are complex processes crucial to wound healing. The formation of a blood clot is normally the result of tissue injury which initiates the platelet adhesion/aggregation and the coagulation cascade and has the effect of slowing or preventing blood flow in wound healing. However, in certain disease states the formation of blood clots within the circulatory system reaches an undesired extent and is itself the source of morbidity potentially leading to pathological consequences. Circulating platelets become adherent and form an occlusive thrombus either by exposure to atherosclerotic lesions following plaque rupture or in response to pathological shear stress. Many adhesive proteins and various receptors are involved in this complex progress.

One important adhesive plasma protein is the van Willebrand Factor (vWF), a multimeric glycoprotein with a mature subunit of 2050 amino acids. The sequence of vWF is known in the art and can be publicly gained e.g. http://www.ensembl.org/index.html

See for vWF:

http://www.ensembt.org/Homo_sapiens/Search/Resutts?species=Homo_sapiens;idx=;q =vWF
http://www.ensembl.org/Homo_sapiens/Transcript/ProteinSummary?db=core;g=ENSG0 0000110799;r=12:6058-040-6233836;t=ENST00000261405 The sequence of the gene X is known in the art. The coding polynucleotide sequences the vWF are publically available at the NCBI nucleotide database under accession number NM_000552.3. The protein sequence of vWF is publically available at the NCBI protein database under accession number NP_000543.2. NCBI is the national centre for biotechnology information (postal address: National Centre for Biotechnology Information, National Library of Medicine, Building 38A, Bethesda, MD 20894, USA; web-adress: www.ncbi.org), see also (http://www.ncbi.nlm.nih.gov/pubmed/3524673). The cloning of vWF and its amino acid sequence is furthermore published in Biochemistry. 1986 Jun 3;25(11):3171-84.:Amino acid sequence of human von Willebrand factor, Titani K, KumarS, Takio K, Ericsson LH, Wade RD, Ashida K, Walsh KA, Chopek MW, Sadler JE, Fujikawa K.)

Two platelet membrane glycoprotein receptors for vWF have been identified. Unactivated platelets bind vWF through the platelet GPlb complex. This interaction is induced physiologically by high shear or by binding of vWF to any surface. Subsequently vWF changes the conformation and presents the A1 binding domain in such a way that interaction becomes possible. After activation, platelets express a second binding site for vWF, the GPIIb-IIIa complex, which is also a binding site for fibrinogen. Platelet activation induces amplification mechanisms which finally lead to a firm platelet attachment.

The essential role of GPlb in platelet adhesion was established with the use of antibodies and by observations on a genetic defect the Bernard-Soulier syndrome in which GPlb is absent from platelets. Platelets from Bernard-Soulier patients poorly adhere and moderately aggregate in response to vWF. Also a lot of snake venom proteins are reported which modulate the interaction ob GPlb and vWF.
Specific inhibition of the interaction of GPlb to vWF using monoclonal antibodies or snake venom proteins is an effective means of controlling thrombus formation caused by arterial injury or thrombotic complications. There is also experimental evidence suggesting that small peptides are efficient inhibitors of the GPlb-vWF interaction (Benard, S.A., Smith, T.M., Cunningham, K., Jacob, J., DeSilva, T., Lin, L., Shaw, G.D., Kriz, R., Kelleher, K.S., Biochemistry 47 (16) 4674, 2008). There is also experimental evidence suggesting that inhibition of the GPlb-vWF interaction inhibits thrombus formation with a wider safety window than abciximab an antibody for GPIIb-IIIa which is already launched (Kageyama, S.; Yamamoto, H.; Nakazawa, H., Yoshimoto, R. Thromb. Res. 101 (2001) 395-404). However there is still no drug available for patients to inhibit the GPlb-vWF interaction and thus inhibit thrombus formation.

With a continued need for safe and effective therapeutic antithrombotic agents to limit or prevent thrombus formation, it is very desirable to screen for novel agents, such as agents able to separate - as far as anyhow possible - a reduction in thrombotic events from an increase in bleeding events. One approach being the search for agents that target an early step in thrombogenesis like inhibition of the GPlb-vWF interaction. Moreover, there are many other platelet surface molecules involved in the interaction of platelets with other molecules and/or the activation of platelets such as proteinase-activated receptor 1 (PAR1) platelet glycoprotein VI (GPVI), integrin $\alpha 2\beta 1$, 5-hydroxytryptamine receptor 2A ($5HT_{2A}$) and prostaglandin E2 receptor EP3 subtype ($EP_3$), etc. that would be desirable targets for new and safe antithrombotic therapies.

Thus, there is a large need for specific modulators, especially inhibitors of the interaction of platelets or platelet surface molecules with binding partners (mostly receptors or ligands).

State of the art screening methods for platelet inhibitors mostly assay the ability of platelets to adhere to certain interaction partners (such as fibrinogen) under shear stress or under static conditions (see e. g. Blue et. al." Application of high-throughput screening to identify a novel αIIb-specific small-molecule inhibitor of αIIb3-mediated platelet interaction with fibrinogen", Blood, p, 1. February 2008, Volume 111, Number 3, p.1248-1256 (2008), the disclosure of which is to be incorporated in present specification). These methods however mostly fail to identify toxic effects induced by the tested test substances. Thus, many of the active test substances identified with state of the art high throughput assays have toxic and other unwanted effects, thus necessitating large and costly secondary profiling or counter-screening campaigns to discriminate modulators exhibiting said unwanted properties.

It is thus the object of present invention to provide screening methods of platelet modulating test substances that are fast and allow for the identification of unwanted side effects, such as platelet toxicity. This object is solved by the methods according to present invention.

The present invention satisfies the above needs by providing an improved assay method for the identification of novel test substances modulating the interaction of platelets or plateles surface molecules, such as GP1 b with their interaction partners, such as vWF wherein unwanted side effects, such as platelet toxicity of the test substance can be monitored in parallel.

According to one embodiment, the present invention relates to a method for detecting and/or quantifying the interaction of platelets or a platelet surface molecule with an interaction partner comprising the steps of

    (a) providing a multiwell plate amenable for optic analysis by laser scanning cytometry or microscopy or by fluorescence cytometry or microscopy, wherein one or more wells of the plate are coated with an interaction partner of a platelet surface molecule
    (b) bringing into contact of labeled platelets with

        i) one or more interaction partner -coated wells and one or more of ii, iii or iv:
        ii) one or more wells (of the above or an additional multiwell plate) coated with one or more molecules known not to interact specifically or strongly with platelets or platelet surface molecules (low control)
        iii) one or more wells (of the above or an additional multiwell plate) coated with one or more molecules known to specifically interact with platelets or platelet surface molecules known not to specifically or strongly interact with and/or activate platelets (high control)
        iv) one or more wells (of the above or an additional multiwell plate) coated with one or more molecules known to bind but not to activate platelets (activation control), wherein the molecules according to III and Iv can be the same

    (c) washing the wells to remove unbound platelets (i.e. platelets that did not interact specifically with the interaction partner)
    (d) detecting by laser scanning or fluorescence microscopy or cytometry in a pre-selected area of the wells and/or of the plate (wherein a part or the whole area of the plate and/or of each well can be analysed)

        i) the total number of platelets (wherein the number of platelets in bi) divided by the number of platelets in bii) multiplied by 100 gives the percentage X of platelets specifically bound to the interaction partner) and/or
        v) the number of activated platelets, wherein the number of platelets in either b i), ii), iii) or iv) divided by the number of activated platelets in the same of b i), ii), iii) or iv) multiplied by 100 gives the percentage of activated platelets in each of b i), ii), iii) or iv), and

    wherein interaction partners with strong platelet interaction can be identified based on a high number of detected platelets in comparison to the low control and or a high number of activated platelets in comparison to the low control, whereas partners with weak or low platelet interaction can be identified based on a low number of detected platelets in comparison with the high control and/or a low number of activated platelets in comparison to the high control.

Interaction partners with strong platelet binding interaction can be identified based on e.g.

- A high number of platelets and/or
- A high number of activated platelets

Interaction partners with weak or low platelet binding interaction can be identified based on

- A low number of platelets and/or

- A low number of activated platelets

This method according to present allows for the identification of new platelet-interaction partners, as well as the analysis of known platelet-interaction partners, wherein - depending on the parameters to be included into the analysis, also effects besides platelet-interaction can be easily and quickly detected (such as platelet toxic effects, platelet activation etc). The platelet interaction partners to be analysed can be any kind of molecule or molecule complex as well as mixtures of more than one molecule and can also be any kind of test substance or mixtures comprising one or more test substances as defined below.

Towards the end of analysing and detecting such additional effects, one or both of the following parameters can be determined:

a) the number of platelets with weakened fluorescence signal
b) the number of platelets with aberrant morphology (fragments)
c) the number of objects with aberrant, red-shifted fluorescence (large red material), and
wherein a high number in either of a, b or c detected for b i) in comparison to b iii or iv) (the high or activation control) acts as an identifier for toxic or aberrant effects of the interaction partner.

Another aspect of present invention refers to a method for detecting and/or quantifying the capability of a test substance to modulate the binding of a platelet or platelet surface molecule with an interaction partner comprising the steps of:

(a) providing a multiwell plate amenable for optic analysis by laser scanning or fluorescence cytometry or microscopy, wherein one or more wells of the plate are coated with an interaction partner of a platelet surface molecule
(b) bringing into contact of labeled platelets with

i) one or more interaction partner -coated wells and optionally with one or more of ii, iii or iv:
ii) one or more wells (of the above or an additional multiwell plate) coated with one or more molecules known not to interact specifically or strongly with platelets or platelet surface molecules (low control)
iii) one or more wells (of the above or an additional multiwell plate) coated with one or more molecules known to specifically interact with platelets or platelet surface molecules known not to specifically or strongly interact with and/or activate platelets (high control)
iv) one or more wells (of the above or an additional multiwell plate) coated with one or more molecules known to bind but not to activate platelets (activation control) III and Iv can be the same

(c) bringing into contact of a test substance with the platelets, (wherein this can occur prior, after or at the same time (together) with step b))
(d) washing the wells to remove unbound platelets
(e) detecting by laser scanning or fluorescence microscopy or cytometry in a pre-selected area of the wells or the plate the total number of platelets and optionally
(f) detecting by laser scanning or fluorescence microscopy or cytometry in a pre-selected area of the wells one or more of the following parameters I, ii, iii or iv:

i) the number of platelets with weakened fluorescence signal
ii) the number of platelets with aberrant morphology (fragments)
iii) the number of objects with aberrant, red-shifted fluorescence (large red material),
iv) the number of activated platelets, and

(g) comparing the values according to (e) and optionally (f) with one or more reference values generated without addition of test substance,
wherein a difference between the values detected with and without test substance indicates that the test substance is capable of modulating the binding of the platelet surface molecule or of platelets.

A substance / test substance / active substance as to be employed for the different aspects of present invention can be any biological or chemical substance or natural product extract, either purified, partially purified, synthesized or manufactured by means of biochemical or molecular biological methods. It can be any chemical or biological molecule or molecule complex, such as, but not limited to: a chemical test substance or small molecule compound (such as an organic molecule), a nucleic acid (such as DNA, RNA, long or short, such as oligonucleotide, siRNA etc.), protein (e.g. peptide, antibody in any kind of known format mono- dual- or multispecific or fragment thereof, or other protein-binding entity such as proteinaceous scaffolds (e.g. Darpin, Ankyrin)) etc.

If in the method for the detection or quantification of the capability of a test substance to modulate the binding of a platelet surface molecule with an interaction partner, one or more of f) i, ii or iii or is detected, then an increase between one or more of the values of f) i, ii, iii or iv) in comparison to a reference sample without test substance is indicative of toxic effects of the test substance.

Moreover, if in such a method the number of activated platelets according to f) iv is detected, then an increase of the number of activated platelets in comparison to a reference sample without test substance is indicative of platelet-activating effects of the test substance.

The total number of platelets can be identified by one, two or three of the following parameters

- the fluorescence intensity being between minimum and maximum value as measured in individual, isolated platelets adhering to the interaction partner of interest,
- the size of the individual isolated platelets between a pre-selected minimum and maximum value (e.g. in comparison to average parameters of the high control) measured in platelets adhering to the interaction partner of interest,
- the emitted wavelength spectrum ("color") of the detected fluorescence being between a minimum and maximum value (e.g. in comparison to average parameters of the high control) measured in platelets adhering to the interaction partner of interest.

The precise spectrum, as well as the fluorescence intensity and the size are dependent on the spectral properties of the fluorophore used for staining the platelets and the experimental settings (type of detector used; the wavelengths used for excitation and for emission ), their choice is known to the skilled artisan.

The number of platelets with weakened fluorescence signal ("dim platelets"), is indicative of platelet toxic effects of an analyte (a test substance to be analysed). This can be identified e.g. by one, two or three of the following parameters:

- the mean and/or peak fluorescence intensity per single platelet being lower than the mean value measured for single platelets with regular fluorescence intensity (such as those of the high control or any other control sample without test substance addition) adhering to the interaction partner of interest. Preferably, the mean and/or peak fluorescence intensity per platelet is 90% or lower, 80% or lower, 70% or lower, 60% or lower or 50% or lower than that of the mean value for platelets with regular fluorescence intensity, with 70% or lower being preferred.
- the size of the individual, isolated platelets being between minimum and maximal value as measured in single platelets adhering to the interaction partner of interest (see also above),
- the emitted wavelength spectrum ("color") of the detected fluorescence being equal to that of platelets adhering to the interaction partner of interest. The precise spectrum is dependent on the spectral properties of the fluorophore used for staining the platelets (see below) and the experimental settings (type of detector used; the wavelengths used for excitation and to detect the emitted fluorescent light).

The number of platelets with aberrant morphology fragments (debris), as indicative of toxic effects of the analyte (test substance to be analysed) can e.g. be identified by means of one, two or three of the following parameters:

- the size of the individual objects being lower than the mean value as measured in single platelets with regular morphology adhering to the interaction partner of interest (e.g. as in comparison with the mean value of size of individual platelets of the high control or any other control sample without test substance addition). Preferably, the size per individual platelet is is 90% or lower, 80% or lower, 70% or lower, 60% or lower or 50% or lower than that of the mean value as measured in single platelets with regular morphology adhering to the interaction partner of interest, with 70% or lower being preferred.
- the mean and/or peak fluorescence intensity per single object being 1% -100%, 2% -100%, 3,% -100%, 4% - 100%, 5%- 100%, 6% - 100%, 7% -100%, 8% - 100%, 9% -100 % or 10% - 100 % of the values measured in platelets with regular morphology adhering to the interaction partner of interest, with values from 5%-100% being preferred.
- The emitted wavelength spectrum ("color") of the detected fluorescence being equal as measured in platelets (with regular morphology) adhering to the interaction partner of interest. The precise spectrum is dependent on the spectral properties of the fluorophore used for staining the platelets (see below) and the experimental settings (type of detector used; the wavelengths used for excitation and to detect the emitted fluorescent light).

The number of objects with an aberrant, red-shifted fluorescence (large red material), as indicative of platelet toxic effects of an analyte (the test substance to be analysed), can e.g. be identified by means of one, two or three of the following parameters:

- The emitted wavelength spectrum ("color") of the detected fluorescence of the objects of shifted to longer wavelengths compared to the maximum wavelength measured in platelets adhering to the interaction partner of interest. The absolute value is dependent on the experimental settings (type of detector used; the wavelengths used for excitation and for emission).

- The size of the individual objects being approximately 5-500 $\mu m^2$, approximately 7-400 $\mu m^2$, approximately 8-350 or approximately 10 - 300 $\mu m^2$, with a size of approximately 10 - 300 $\mu m^2$ being preferred. These values are dependent on the type of detector used; the wavelengths used for excitation and for emission.

The number of activated platelets, can e.g. be identified by means of one or two or three of the following parameters:

- The size of the object, consisting of groups of platelets, being above the mean maximum value measured for single platelets adhering to the interaction partner of interest (in a control without addition of platelet activating activity, e.g. in the high control or the activation control),
- The mean fluorescence intensity and mean fluorescent peak intensity being between a minimum and maximum value as measured in platelets adhering to the interaction partner of interest (e.g. the high control),
- The emitted wavelength spectrum ("color") of the detected fluorescence being between the mean maximum and minimum value as measured in platelets adhering to the interaction partner of interest (e.g. in the high control) The precise spectrum is dependent on the spectral properties of the fluorophore used for labelling the platelets (see below) and the experimental settings (type of detector used; the wavelengths used for excitation and to detect the emitted fluorescent light).

Based on the measured data, the following calculations can be made:

- % of one population (X) to another population (Y) =

$$\% = \left( \frac{\text{Number of objects in population X}}{\text{Number of objects in population Y}} \times 100 \right)$$

The percentage of inhibition of a measured feature/parameter can e.g. be calculated by

$$\% \text{ inhibition} = 100 - \left( \frac{\text{Value of feature detected in compound-treated well (well coated with high-binding interaction partner ) minus value of feature detected in low control (well coated with non or low-binding interaction partner )}}{\text{Mean value feature detected in high control (well coated with high-binding interaction partner )I minus mean value feature detected in low control (well coated with non or low-binding interaction partner )}} \times 100 \right)$$

The percentage of activation of a certain feature/parameter can e.g. be calculated by:

$$\% \text{activation} = \left( \frac{\text{Value of feature detected in compound-treated well (well coated with high-binding interaction partner )}}{\text{Mean value feature detected in high control (well coated with high-binding interaction partner )I}} \times 100 \right)$$

Analysing in a pre-selected (at once) area guarantees that the detected values are comparable between the different samples. This pre-selected area can be a determined area of the plate comprising one or more whole wells or it can be a pre-selected area within one well or a combination of both (e.g. a pre-selected area of the plate, wherein in each well a pre-selected area of the well is analysed), the pre-selected area can also be the whole of the plate (e.g. all wells of the plate) and/or the whole of each well. In one embodiment the area to be analysed is 3-5mm x3-5 mm, in another embodiment it is 3x3mm, 4x4mm or 5x5mm with 4x4 mm being preferred, moreover the pre-selected area can be a defined number of wells per plate (e.g. 10-20, 14-17, 14, 15,16, or 17). If only some of the wells of a plate is analysed at the same time, then this step can be repeated with other wells of the plate or another part of the plate is analysed in a different step of one of the methods according to the invention, until all method steps have been performed.

The populations described above are e.g. measured in the following wells:

- High-binding control: one or more wells (preferably a defined number) coated with an interaction partner that is known to specifically interact with platelets and/or platelet surface molecules (e.g. a high-binding interaction partner with relatively strong interaction like vWF-A1 or collagen1)
- Non-binding or low-binding control is one or more wells (preferably a defined number, e.g. such as the same number as for the high binding control) coated with a non-interaction or a low-interaction partner (e.g. a molecule or molecule complex, test substance etc. that is known not to specifically or strongly bind and interact with platelets or platelet surface molecules), like BSA.
- Experimental wells: One or more wells (preferably a defined number, e.g. such as the same number as for the high and/or the low binding control) coated with the interaction partner of interest, wherein the test test substance can be added to these wells added at the same time, prior or after platelet addition, with addition prior to platelet addition being preferred.

As detailed later in the examples section, according to one example, 16 wells per reaction were analysed for each of the above 3 well-types (high control, low control and experimental well).

Test substances that reduce platelet interaction with a binding partner and/or reduce platelet activation can be identified by e.g the following parameter:

- The value for the percentage inhibition of the feature "number of platelets" being above the mean value of all high-binding control control wells.

Test substances that increase platelet interaction with a binding partner and/or increase platelet activation can be identified by e.g. the following parameter:

- The value for the percentage inhibition of the feature "number of platelets" is above the mean value obtained for all experimental wells. or above the mean value of all Non-binding or low-binding control wells.

Test substances that have a toxic effect on platelets can e.g. be identified based on one or two or three of the following parameters

- A decrease in the total number of platelets: The value of the percentage inhibition of the feature "number of platelets" in the experimental wells for a given test substance is above the mean value of all experimental wells. or above the mean value of all High-binding control wells.

- An increase of platelet debris: The result of the following calculation - percentage of the feature "number of platelets with aberrant morphology fragments (debris)" divided by the percentage of the feature "number all platelets" - lies above the mean value obtained for. or above the mean value of all High-binding control wells.

- An increase of dim platelets: The result of the following calculation - the value for the percentage of the feature "number of platelets with weakened fluorescence signal ("dim platelets")" divided by the percentage of the feature "number of platelets" - lies above the mean value for or above the mean value of all High-binding control wells.

Each of these three parameters analysed alone or any other combination with other parameters gives reliable indication regarding the toxicity of a test substance.

The feature combination "decrease total number of platelets" with "increase of platelet debris" is a very reliable parameter combination for determination of toxic effects. If, the "increase of dim platelets" is analysed in addition, this leads to yet more reliable results.

Test substances that increase platelet activation can be identified e.g. based on one or both of the following parameters:

- An increased number of activated platelets: The value of the percentage of the feature "number of activated platelets" divided by the percentage of the feature "number of platelets"; this value is above the mean value for all experimental wells

- The value for the percentage of the feature "number of objects with an aberrant, red-shifted fluorescence (large red material)" divided by the percentage of the feature "number of platelets"; this value is above the mean value obtained for all experimental wells

Test substances that decrease platelet activation can be identified e.g. based on one or both of the following parameters:

- A decreased number of activated platelets: The result of the following calculation

    - the percentage of the feature "number of activated platelets" divided by the percentage of the feature "number of platelets"; this value is below the mean value for all experimental wells

- A decreased amount of large red material: The result of the following calculation

    - the value for the percentage of the feature "number of objects with an aberrant, red-shifted fluorescence (large red material)" divided by the percentage of the feature "number of platelets" - is below the mean value obtained for all experimental wells

As stated above, the platelets can be brought into contact with the test substance before, after or at the same time as the bringing into contact of the platelets with the plate; according to one example, the platelets are mixed with the test substance prior to bringing into contact of test substance and platelets with the coated plate.

The platelets can be labelled with any marker that allows for a detection by laser scanning and/or fluorescence microscopy or cytometry. Examples of markers include fluorescent dyes such as: calcein-AM (e.g. such as available at Invitrogen.com), CMFDA (5-choromethylfluorescein diacetate (e.g. such as available atInvitrogen.com), , PKH67 (e.g. such as available at Sigma-Aldrich.com), resazurin (e.g. such as available at Invitrogen.com), Lavacell (e.g. such as available under the following link: http://www.activemotif.com/catalog/116/lavacell-live-cell-membrane-staining.html), (fixable) FM1-43 (e.g. such as available at Invitrogen.com), Nano-Orange (e.g. such as available at Invitrogen.com) or fluorescently-labelled phalloidin (e.g. such as available at Invitrogen.com)

The platelet surface molecule can be any surface molecule of blood platelets (e.g. human or non-human platelets) such as a protein or glycoprotein, such as a receptor or other platelet surface protein, e.g. GP1b, the collagen receptor, PAR1, PAR4, P2Y purinoceptor 1, P2Y purinoceptor 12, junctional adhesion molecule A (JAMA), JAMC or any other known platelet-surface molecule.

The interaction partner of the platelet or platelet surface molecule can be a biological or an artificial (e.g. chemical) molecule or supramolecular complex interacting with a platelet or platelet surface molecule, e.g. an artificial or naturally occurring ligand of a platelet surface molecule or receptor or a functional fragment or derivative thereof (wherein the fragment or derivative is considered to be functional if it is still able to interact with the surface molecule or receptor that the naturally occurring ligand interacts with).

Examples of interaction partners include the van Willebrand factor 1 (vWF-1), the A1 domain of vWF-1, collagen, fibrinogen, Protein c, Mac-1 ($\alpha$Mp2), P-selectin, (CD62P), High-molecular weight kininogen, Thrombospondin-1, soluble GPVI), PSGL1, or a functional fragment or derivative of any of these.

According to one example, the interaction partner is vWF-1. According to another example, it is vWF-1 with 95%, 96%, 97%, 98%, 99% or 100% sequence identity to SEQ ID NO:2 or encoded by a nucleic acid comprising a sequence with 95%, 96%, 97%, 98%, 99% or 100% sequence identity to SEQ ID NO:1.

The multiwell plates used in the context of present invention can be any multiwall plate amenable for optic analysis with a laser scanning microscope or fluorescence microscope or with a laser scanning cytometer or a fluorescence cytometer. Such plates are known in the art. Suitable formats include, e.g. a 96-well, a 386-well or a 1536-well plate and preferably a 386-well plate.

Present set of invention also relates to an article of manufacture comprising a leaflet with instructions one or more of the above methods.

In the following, present invention is described in more detail in form of examples which are not to be understood as limiting.

Legend to the figures:

**Figure 1:** Morphology of platelets observed in a microtiter plate after calcein AM labeling.

**Figure 2:** Determination of the optimal amount of vWF-A1 in for platelet adhesion.

Wells were coated with isolated vWF-A1 (white bars), uncoated (blue bar) or BSA coated (black bar). Z' values were calculated using the number of detected platelets in wells coated with A1 as high control vs the number of platelets in BSA-coated wells as low control.

**Figure 3:** Morphology of the platelets after dispension into wells coated with either vWF-A1 (high control (HC) left) of with BSA (low control; LC, middle) or high control (HC) with the GPIb-specific inhibitor OS-1 (right). Pictures were taken in an Olympus IX81 microscope using 60x or 100x objectives with appropriate filter.

**Figure 4:** Well view of a 384-wells plate as generated by the Acumen of a low control well (coated with BSA only) after washing unattached platelets. In some corners of the well more material stayed attached indicated by arrows.

**Figure 5:** Different wash programs, used to remove the unattached platelets, have a profound effect on the S/B and the statistics of the assay. Wells were coated with isolated A1 ("high control"; white bars), uncoated (blue bar) or BSA coated ("low control"; black bar). Z' values were calculated using the number of detected platelets in wells coated with A1 as high control vs the number of platelets in BSA-coated wells as low control.

**Figure 6:** Using an optimized washer program to remove the unadhered platelets, a S/B (number platelets attached to vWF-A1 vs BSA) of almost 70x could be obtained with a Z' value of > 0.6. Under these condition, an almost complete inhibition with OS-1 (a peptide that binds to the GPIb and blocks its interactions with VWF-A1) and with AK2 (a mouse monoclonal antibody against GP-Ib) is obtained.

**Figure 7:** Effects of the amount of vWF-A1 used for coating on number of attached platelets (white bars) and the calculated IC50 values of OS-1 (green triangles).

**Figure 8:** Stability of the plates. Effect of a prolonged test substance incubation (green vs blue histograms shades) and the stability of the plates between the finals wash steps and plate reading (intensity of the green or blue colors).

**Figure 9:** S/B, Z' and inhibition by OS-1 values of platelet - VWF-a1 interaction during a fully automated run of 30 plates. Only the data of the first, 6th, 12th, 18th, 24th and the last plate were shown.

**Figure 10:** Effect on test substances with known toxicity on platelet-A1 interaction.

**Figure 11:** S/B and Z' values of the platelet interaction to collagen 1, using BSA as control. Using the optimized conditions, a specific small test substance inhibitor and an antibody against the collagen recptor (ASC-2) were able to block the adhesion of platelets to levels comparable to control (BSA) values.

**Figure 12:** Z' and S/B values of 6 test plates of the platelet - collagen 1 interaction assay

**Figure 13:** Distribution of the positives of a test substances training set of 69 test substances composed of several subgroups.

**Figure 14:** Morphology of platelets adhering to wells coated with vWF-A1 in absence (left) or in presence (right) of a toxic test substance. In most wells where platelets had been incubated with test substances with cell toxic features, many small green fluorescent objects were observed. Pictures were taken on an Olympus IX81 microscope using 60x objective and a 472/30 nm filter for exitation and a 520/35 nm filter to detect the emitted light.

**Figure 15:** Identification toxic test substances. In wells treated with toxic test substances (C and D) small fluorescent material could be detected instead of the typical green fluorescent platelets observed in well coated with vWF-A1 (B). In addition, platelets with a normal morphology were less frequent in comparison with control wells coated with BSA (A). These small objects were marked in like to make them more clearly. In some wells where platelets were incubated with toxic test substances, the platelets were dimmer (see C).

**Figure 16** shows the boxplot of the feature "median of the mean intensity of green emitted light of objects identified as platelets" (for definition platelets see section "Acumen reading") for its ability to discriminate between different treatments, depicted on the X-axis. The categories High and Low represent the neutral (high) - and stimulator (low) control group respectively.

**Figure 17** shows a correlation plot of all (43) possible features. The colour code indicate the degree of correlation (green is -1, black ~ 0 and red 1).

**Figure 18** shows the result of the importance analysis of the most relevant features in respect of the constructed classifier.

**Figure 19** shows the first three components of singular value decomposition. The second component mainly separates the neutral and stimulator controls. The positive OS1 reference control is clearly scattered in the werea of the stimulator control. Most of the putative toxic test substances were dispersed around the werea of the neutral control. The test test substances spread around 0.025 > second component < 0.075 might be detected as potential inhibitors although they were similar to potential toxic test substances.

**Figure 20** illustrates the result of the hierarchical cluster analysis of putative toxic test substances in respect to the stimulator - and neutral control state. Based on the cluster analysis the putative toxic test substance groups 3 (blue), 5 (orange) & 6 (brown) have been selected for the construction of a classifier.

**Figure 21** illustrates selected groups (neutral - , stimulator control, OS1 reference, tree different tool test substances and three putative toxic test substances). For each single group the 12 dimensional data vector is represented as a star plot. The height of each segment represents the scaled normalised value of the corresponding measurement.

Examples:

Introduction:

Multi parameter analysis of vWF-GP1b interaction in live platelets:

The invention is illustrated in the following in the form of a platelet-based assay, suitable for HTS, for the specific and quantitative measurement of the interaction of live, human platelets with immobilized proteins. This is shown for two proteins that are adhesive to platelets; collagen 1 and the A1 domain from von Willebrand factor. The platelets were dispensed into ca 50 000 multiplate wells and the the unbound platelets removed by washing. The assay was suitable for screening ca 50 000 wells/day. This assay allows for the identification of inhibitors and/or activators, including the acquisition and analysis of multiple cellular parameters hinting towards toxic and platelet activating effects.

Under physiological conditions, platelets aggregate to seal leaks at sites of vascular injury. Plasma von Willebrand factor (vWF) acts as an extracellular adapter in this process, binding to collagen in the wall of damaged blood vessels and then via its A1 domain to membrane glycoprotein Ib complex on the platelet surface. vWF and platelets do not readily interact in the blood, since the vWF-A1 domain is only exposed when vWF binds to collagen at the relatively high fluid shear rates. To translate this event into a biological relevant screening campaign aimed to indentify test substances that inhibit the interaction of von Willibrand factor (vWF) and the GPIb receptor complex, a new binding assay has been developed. In this binding assay, life platelets are added to the wells of a 384-multiwell plate coated with isolated vWF-A1 domain.

The assay protocol was validated with known inhibitors of the vWFA1 protein-protein interaction as tool test substances and a selected number of known cytotoxic test substances (61 in total). The data was further used to find the most important read-out parameters to distinguish between specific and unspecific effects such as cytotoxicity.

Despite the limited stability of the platelets in suspension, a throughput of ca 48 000 single point detections per week was obtained, with a good statistical quality (0.65< Z' < 0.85). The assay was used to set of 619,569 test substances at 10 $\mu$M. The resulting 6,847 positive test substances (based on number of adhered platelets) were reconfirmed and counter screened for inhibition of the interaction between platelets and collagen1, using a similar protocol. From the remaining test substances, $IC_{50}$ values were determined for both the inhibition with both vWF-A1 as well as with collagen1 interaction. 85 test substances were selected as specific actives with high inhibition of platelet interactions to vWF-A1 with low inhibition to collagen. Some of these actives showed an increased binding of platelets to A1 in comparison to collagen for several concentrations.

Circulating platelets must be maintained in a non-reactive state until needed for haemostasis. The endothelial cells of intact vessels prevent blood coagulation, mainly by forming a barrier between platelets and the subendothelial connective tissue. In the subendothelial tissue, there are proteins, like collagen, fibronectin and laminin that are adhesive substrates for platelets. Other modulators of blood clotting are located in the blood stream, a typical example is the von Willebrand factor (vWF). vWF is a multimeric protein that mediates the adherence of platelets to damaged surface. It contains several domains, like A1 and A3 domains in the central portion of the protein that play an important role in trapping and activation of platelets for clotting. Binding sites for platelet vWF-receptor lie within the A1 domain, whereas its homologous A3 domain binds to collagen. In normal circulation, the A1 domain is hidden. Therefore, in the absence of injury, VWF does not appear to interact with circulating platelets. The immobilization by binding of vWF to collagen via its A3 domain also induces a change in confirmation leading to an increased binding affinity for the vWF-receptor on the platelet membrane, platelet attachment, platelet shape change and spreading along the exposed subendothelium, release of exocytosis of platelet granules that leads to recruitment of other platelets and aggregation via activation of the platelets.

In addition, the affinity of platelets to vWF increases dramatically under conditions of high fluid shear stress, conditions that are capable of unfolding VWF and exposure of vWF-A1 sequences for interaction with the vWF-receptor on the platelet. The unfolding process is reversible, without hydrodynamic shear force the protein immediately relaxes back to its compact state. As conditions of high-shear stress are cumbersome to reproduce in the laboratory, especially during high-throughput screening, the A1 domain instead of the whole vWF was used in present assay.

To detect inhibitors for the interaction between vWF-A1 domain and its receptor it is advantageous to use whole platelets instead of the isolated Gplb protein, the protein directly interacting with the vWF-A1 domain (alternatively also referred to as "A1" in the following). The GP1 b protein is part of a complex consisting of GPIb-IX-V. Part of this complex appears to be associated with lipid rafts and there is evidence for a physiological role for this association in GPIb-IX-V-mediated platelet function. Since the localization of the Glycoprotein Ib-IX-V Complex to lipid rafts is required for platelet adhesion and activation, it is plausible that the whole GPIb-IX-V complex is needed for proper activation and signalling (Shrimpton, The Journal of Experimental Medicine, Volume 196, Number 8, 1057-1066)

The importance of the VWF- platelet interaction is clear from quantitative or qualitative defects of the vWF-receptor or vWF protein itself, both resulting in bleeding disorders, with a prevalence of 0.1-1%. Depending where the defect is, several diseases are known; von Willebrand factor disease (vWD) when there are abnormalities in the vWF) and the Bernard-Soulier syndrome or Giant Platelet Syndrome , when there are defects in vWF-receptor.

When platelets interact with a diseased vessel, this same process as described above may result in pathologic vascular occlusion which is responsible for cardiovascular diseases, the major cause of death in the world. The pathophysiological basis for acute coronary syndromes (ACS) is an acute plaque rupture with subsequent platelet adhesion, aggregation and formation of a thrombus. One approach is to inhibit the first step in thrombosis, i.e., platelet adhesion instead of platelet activation or aggregation.

Almost all ACS result from thrombus formation in preexisting coronary atherosclerosis. As a result of plaque rupture the platelets are exposed to the subendothelial matrix that leads to local thrombus formation. This subsequently leads to coronary artery occlusion and acute myocardial infarction (AMI). In post-AMI patients, high-shear-induced platelet adhesion and aggregation is increased and can be inhibited by administration of an anti-vWF-antibody. Therefore therapies that specifically inhibit VWF - platelet interactions are of particular interest as potential new antiplatet drugs (Drugs of the Future 2003, 28(1): 61-67, the role of VWF-collagen interaction in acute platelet thrombus formation, K. Vanhoorelbeke and H. Deckmyn).

The binding of washed platelets to immobilized proteins was determined by imaging the wells of 384 multiwell plates after removing unbound platelets by washing. The aim of this assay was to identify small molecules for their ability to inhibit the interaction between the immobilized protein and the platelets. However, the assay is also amenable for the analysis of biological molecules or supramolecular complexes.

Test substances acting as e.g. specific platelet-A1 interaction inhibitors should decrease the attachment of platelets to A1 coated-wells, without being cytotoxic.

Platelets, obtained from platelet concentrates intended for transfusion purposes, were fluorescently-labelled by calcein-AM (the acetomethoxy derivate of calcein, able to enter live cells and platelets) and added to the wells of a multiplate coated with isolated vWF-A1 domain or serum albumin as control. After incubation, the unbound platelets were removed by washing. The amount of platelets bound to the immobilized vWF-A1 was determined via imaging using a laser-scanning cytometer. Several features were measured, like the number of certain objects, their size, roundness, emitted colour and fluorescent intensity. These parameters were selected by their ability to discriminate test substances of a training set, composed of known inhibitors, toxic test substances and inactive "neutral" test substances. The adhered objects were identified by a combination of these parameters and in this way platelets can differentiated from other blood products and quantified. The developed assay is intended for screening, a throughput of 4 x 30 plates (à 384 wells) per week could be obtained with good statistics (Z' > 0.8) and identified > 90% of the tested 22 reference test substances with sufficient sensitivity.

The aim of this assay was to identify small molecules for their ability to inhibit the interaction between platelets and vWF-A1 domain. These test substances were added to the coated wells just before the labeled platelets. During the development of the assay, it became clear that most test substances, known for their cell toxic features in other cellular assays, were efficient inhibitors of the interaction of platelets with the A1 domain. Via a careful multi-parameter analysis, the cytotoxic test substances could be discriminated from non-toxic inhibitors. Further multi-dimensional analysis also filtered against test substances with toxic effects resulting in an inhibition of adhesion of platelets. Comparison of the structures of the test substances identified structural common elements that are prime candidates for further drug-development.

The assay protocol was validated with known inhibitors of the vWFA1 protein-protein interaction as tool test substances and a selected number of known cytotoxic test substances (61 in total). The data was further used to find the most important read out parameters to distinguish between specific and unspecific effects such as cytotoxicity.

The assay is suitable for screening 600K test substances in 384 format. Despite the limited stability of the platelets in suspension, a throughput of ca. 48000 single point detections per week was obtained, with a good statistical quality (Z' > 0.7). The Validation Collection has been tested in triplicates hinting a hit rate of 0.7- 0.9% (without subtraction of potentially toxic test substances).

High-content screening (HCS)

High-content screening (HCS) allows the detection of whole-cell morphological changes and signaling events that are involved in a number of biological processes, such as protein localization, translocation, phosphorylation, protein-protein interactions and second messenger cascades. Such image-based screening approaches have been called HCS because of their rich information content. Images of cells in microtiter plates are automatically generated and analysed using image analysis algorithms. In this way multiple biochemical and morphological parameters of cells relevant to test substance effects can be the simultaneously measured, like fluorescence intensity, granularity, cell shape, intracellular organelles' organization, spatial relationships of objects of interest. Major benefits of HCS for drug discovery are that it allows: e.g. the combination of parameters ('multiplexing') to define subpopulations of cells or subcellular structures and possibly the early detection of undesirable effects (toxicity).

Drug profiling by comparison with parameters of pharmacological profiles of well-known reference standards: comparing the cellular activity profiles of phenotypic hits to those of known drugs and pharmacological references.

Structure - activity relationsship' of hits by chemical structure analysis: the opportunity of finding test substances that yield similar biological effects as detected by the multi-parameter analysis. Careful analysis of the chemical structures of a collection of test substances with similar activities on cells, allows for the identification of common structural elements. This will enhance simplify the prioritization and speed up lead development by chemical optimization of promising chemical candidates.

Multi-parameter analysis

Generation multiple-parameters from single cell analysis lead to a significant increase in data. Analysis of data in a multidimensional space can be a difficult task, in contrast to the usual rank ordering by efficacy or potency in a single-read-out assay. To take full advantage of this plethora of parameters obtained from HCS the parameters need to be assessed, analyzed and interpreted in a biological, chemical and pharmacological context. Therefore well characterized control and reference states were considered in the course of the analysis of the multidimensional data. Normalising of the multidimensional data set is an important first step. This task was accomplished by multiple commercial and academic software solutions. Due to the fact that well defined reference states such as mode of action of tool test substances or natural ligands, are normally measured independently from the main high throughput screen, it is important to select carefully the appropriate normalisation method. As soon as the multidimensional data was normalised correctly, standard multivariate data analysis techniques such as cluster-, factor analysis, classification- and filtering techniques were applied.

Acumen technology

Acumen is a reader that allows cell-based screening using a 488 nm laser for excitation and simultaneous four colour detection. Acumen technology applies cytometric principles, rather than image analysis. The laser scans the entire field of view collecting intensity readings at regular intervals. This allows imaging the whole well, instead of making images from part of the well as is done by regular microscopy. Whole well analysis eliminates the risk of making false interpretations by analysis images that are not representative for the whole well. Thresholding algorithms identify all fluorescent intensities above the solution background. The Acumen technology is fast and robust results - averaging around 10 minutes per plate for any plate .

1. Materials

1.1 Storage

All materials were stored as recommended by the suppliers, as described in their websites (listed below) or in their product information.

1.2 Cells

Thrombocytes were obtained via the DRK-Blutspendedienst Baden-Wortemberg-Hessen GmbH; Producer: Institut Frankfurt, PF 730367 D-60505 Frankfurt. Transport occurred by http://www.bluttransport.com/angebot/ according to the legal guidelines for bloodproducts. Platelet concentrates were stored at RT under gentle agitation on a TiMix by Edmund Buehler - setting 6. http://www.edmund-buehler.de/english/i-schuettler-und-inkubationshauben.pml. The concentrates were made from 4 pooled donors and contained appr. 30% Plasma.

1.3 Plates

Assay-Plate: Greiner hb (high binding), black, clear bottom, 384-well, Greiner # 781097

1.4 Chemicals

| REAGENT | provider | Order -Nr. | Form MW | Storage |
|---|---|---|---|---|
| Trizma base | SIGMA | T-1503 | solid MW 121,14 | RT |

(continued)

| REAGENT | provider | Order -Nr. | Form MW | Storage |
|---|---|---|---|---|
| Trizma HCl | SIGMA | T-3253 | solid MW 157,6 | RT |
| NaCl | SIGMA | S-3014 | solid MW 58,44 | RT |
| KCl | SIGMA | P-9541 | solid MW 74,55 | RT |
| CaCl2 | SIGMA | C-3306 | solid MW 147,0 | RT |
| MgCl2 x 6H2O | SIGMA | M-2670 | solid MW 203,31 | RT |
| 10% Pluronic-F-68 | SIGMA | P-5556 | liquid MW 8350 | RT |

A commercial solution consiting of 10% Pluronic F-68 in water

| PGE1 | SIGMA | P-5515 | solid | - 20°C |
|---|---|---|---|---|
| Stock made of 0,5mg PGE1 /ml in ethanol absolute , | | | liquid | - 20°C |
| Calcein-AM | Invitrogen | C-3099 | liquid | - 20°C |

**A commercial solution containing calcein-AM** *1 mg/mL (= 1,005 mM) in dry DMSO

| ReoPro | Centocor B.V | PZN 7123941 | liquid 2mg/ml | 2-8°C |
|---|---|---|---|---|

1.5 Proteins and antibodies

| BSA (for Assay-buffer) | | | | |
|---|---|---|---|---|
| | SIGMA | A-7906 | solid | + 4 °C |
| BSA Fatty Acid free (for Blocking Solution) | | | | |
| | SIGMA | A-7030 | solid | + 4 °C |
| vWF-A1 (= von Willebrand Faktor A1 domain (Aa 475-706), | | | | |
| | | | liquid; 5,3 mg/ml | - 80°C |

1.6 Buffers and solutions

TBS- 10X - concentrate = Basis for all subsequent buffers: 500 mM TRIS-HCl, pH7,4, 1200mM NaCl, 27mM KCl, 0,5mM CaCl2; storage RT or +4°C
1 M MgCl2-Stock solution
TBS-vWF-A1 -Coating/Blocking-Puffer 1 X, for vWF-A1 -Coating-solution and for Blocking-solution: 2mM MgCl2 in 1xTBS-buffer
TBS-Adhaesion buffer 1X (=Assay buffer, freshly prepared), used for thrombocyte preparations, for test substance-dilutions and controls: 2 mM MgCl2 and 0.1%BSA (SIGMA - A-7906) in in 1 x TBS
TBS-washbuffer 1X, for washing after coating, blocking, and after assay-incubation Pluronic-F-68 0.001% in TBS-Adhaesionbuffer 1X
vWF-A1-coating solution in TBS-vWF-A1-Coating/Blocking-buffer (1x): 30 μg/ml vWF-A1 in 2mM MgCl2 in 1xTBS-buffer actual batch: 5,3 mg/ml
5% Blocking buffer in TBS-vWF-A1-Coating/Blocking-Puffer 1X : 5% BSA powder, fatty acid free (SIGMA - A-7030)
PGE1 - Assay-concentration: 0,25 μg/ml , store - 20°C
2 μl of the PGE1-Stock-solution (0,5 mg/ml),must be added before 4 ml Thrombocyte-concentrate from DRK / German Red Cross: is added
Calcein-AM - Assay-concentration: 2,5 μM/ml,
5 μl from the Calcein-AM-Stock-solution (1 mg/ml) are added to 2 ml of Thrombocyte suspension, with a concentration of $2^8$ thrombocytes/ml:
ReoPro - Assay-concentration: 2,5 μg/ml, store at +4°C - ready to use;

| | |
|---|---|
| Thrombocyte-solution von 2E 08/ml auf 5E 07/ml | > 1:4 |
| TBS-Adhasions-Puffer pipet first | 3 ml |
| ReoPro-Stock-solution (2 mg/ml) add & vortex briefly | 5 $\mu$l |
| Thrombocyten 2E 08/ml | 1 ml |

Thrombocyte-dilution for 384-Assay (10000 cells/Well in 20 $\mu$l)

| | |
|---|---|
| Thrombocyte-Dilution from 5E 07/ml to 5E 05/ml | > 1 : 100 |
| TBS-Adhasions-buffer pipet this buffer first, add "Flügelrührfisch" (Wing-stirring-bar) to the PP-beaker | 99 ml |
| Thrombocyte 5E 07/ml | 01 ml |

1.7 Reference test substances

a) Two specific GPIb inhibitors were used:

OS-1 (in house synthesis), see Benard et al. Biochemistry, 2008, 47 (16), pp 4674-4682: http://www.ncbi.nlm.nih.gov/pubmed/18363340
The OS-1 peptide binds to the GPIb and stabilizes the GPIb in such a way that it gets incapable of forming key interactions with VWF-A1.

b) Mouse anti-human CD42B monoclonal antibody from Chemicon International. The antibody is directed against the CD42b antigen; platelet glycoprotein Gp1 b alpha, thatserves as a receptor for von Willebrand factor (vWF); clone name: AK2; catalog No. CBL166,
http://www.novaseptic.it/publications.nsf/a73664f9f981 af8c852569b9005b4eee/831 f4ef 5b24d7bb585257-3060071 aabf/$FILE/ATTV23JL/CBL166.pdf

2. Methods

2.1 Coating of the assay plates with vWF-A1 and blocking by BSA

25 $\mu$l of the TBS-vWF-A1-Coating/Blocking-Puffer 1X (containing 15 $\mu$g/ml vWF-A1) is added sterile per well using a Multidrop from Labsystems. Plates were incubated and stored minimally overnight up to one week. Storage is at 4C, sterile and protected against evaporation.
The sequence of the vWF-A1 domain as used herein is: (the A1 domain of human vWF was cloned BamHI/HindiIII into a standard expression vector, expressed and purified with known methods):

cDNA Sequence (SEQ ID NO:1)

```
atgagaggatcgcatcaccatcaccatcacggatcccaggagccgggaggcctggtggtgcctcccacag
atgccccggtgagccccaccactctgtatgtggaggacatctcggaaccgccgttgcacgatttctactg
cagcaggctactggacctggtcttcctgctggatggctcctcaggctgtccgaggctgagtttgaagtg
ctgaaggcctttgtggtggacatgatggagcggctgcgcatctcccagaagtgggtccgcgtggccgtgg
tggagtaccacgacggctcccacgcctacatcgggctcaaggaccggaagcgaccgtcagagctgcggcg
cattgccagccaggtgaagtatgcgggcagccaggtggcctccaccagcgaggtcttgaaatacacactg
ttccaaatcttcagcaagatcgaccgccctgaagcctccgcatcgccctgctcctgatggccagccagg
agccccaacggatgtcccggaactttgtccgctacgtccagggcctgaagaagaagaaggtcattgtgat
cccggtgggcattgggccccatgccaacctcaagcagatccgcctcatcgagaagcaggcccctgagaac
aaggccttcgtgctgagcagtgtggatgagctggagcagcaaagggacgagatcgttagctacctctgtg
accttgcccctgaagcccctcctcctactctgccc
```

Amino acid sequence (SEQ ID NO.2)

```
        10         20         30         40         50         60
MRGSHHHHHH GSQEPGGLVV PPTDAPVSPT TLYVEDISEP PLHDFYCSRL LDLVFLLDGS
        70         80         90        100        110        120
SRLSEAEFEV LKAFVVDMME RLRISQKWVR VAVVEYHDGS HAYIGLKDRK RPSELRRIAS
       130        140        150        160        170        180
QVKYAGSQVA STSEVLKYTL FQIFSKIDRP EASRIALLLM ASQEPQRMSR NFVRYVQGLK
       190        200        210        220        230        240
KKKVIVIPVG IGPHANLKQI RLIEKQAPEN KAFVLSSVDE LEQQRDEIVS YLCDLAPEAP

PPTLP
```

**Number of amino acids: 245**

**Molecular weight: 27683.9**

**Theoretical pl: 7.83**

One hour before test substance addition, the wells of the plates were blocked with BSA. Before the wells were washed with 1 x TBS-Washbuffer , 60 µl / Well using an ELx405 Microplate Washer (96 wells) from Biotek http://www.biotek.com/

Immediately after (wells should not dry out) the wells were blocked 5% BSA-Fatty acid free 40µl/Well using a Multidrop from Labsystems . Wells were incubated 1-2 hours until test substances were added.

2.2 Test substance dilution

Sealed plates delivered from test substance management were stored at -20 °C until use. Test substances were dissolved in 100% DMSO at 3 mM. Test substances were diluted in TBS-Adhaesionbuffer 1X till 10 µM final concentration. Diluted test substance were stored at RT and used within hours. HTS occurred in single-point determination.

2.3 Washing the wells and test substance addition

Surplus of BSA is washed away by washing the wells with 1 x TBS-Washbuffer, 60 µl / Well using an ELx405 Microplate Washer (96 wells) from Biotek http://www.biotek.com/ , followed by the addition of 10 µl test substance solution or high and low control using Cybio robotics http://www.cybio-ag.com/.
Plates were not allowed to dry out.

2.4 Controls during HTS

| | |
|---|---|
| LOW-Control: | no vWF-A1-Coating, just BSA-treated plates for blocking |
| HIGH-Control: | vWF-A1 + |
| Standard (non-toxic GP1b inhibitor) | vWF-A1+ and OS-1 10 µM |

2.5 Platelet labeling and dispensing

The total time needed between removing the platelets from the platelet concentrate, as obtained from the German Red Cross and dispensing the platelets into the the multiwall plates should be between 45 - 60 mins.
The bag containing the the platelet concentrate, as obtained from the German Red Cross, is removed from the agitation table (see 1.2; cells) and is put in a sterile flow cabinet on an absorbing towel to absorb any spills. The tubing, tube clamps and sciccors are s terilized with 70% ethanol in water.
The tubing from the bag with platelet concentrate is clamped and the tubing is cut.
The clamp is released and the first ca. 10 ml concentrate that comes out of the bag is discarded. 30 ml concentrate is poured into n 50ml BD-Falcon tube made of polypropylene. www.bd.com/

Next, 2.5 μl PGE-stock (0.5 mg/ml) is added to an empty 12 ml round-bottom tube made of polypropylene (Greiner, www.greinerbioone.com). To this 5 ml platelet concentrate is added (final concentration of PGE1 is 0,25 μg/ml) and the tube is gently mixed and incubated 5 min at RT.

Next the tube is centrifuged at 300 xg in a Minifuge T **(Heraeus** Holding GMBH, **Hanau)** during 15 min at room temperature, acceleration setting 3 and brake setting 1 After centrifugation, the supernatant is decanted and the pellet is carefully resuspend by "ticking" in TBS-1x,, using first 2.5 ml to resuspend the pellet, next ca 1 - 2 ml, take care that the total volume should not exceed 5 ml.

To determine the number of platelets, 2 ml of this platelet suspension is put into a new tube and diluted with 3 ml Assaypuffer. From this an aliquot of ca. 0,2 - 0,5 ml is pipetted into an Eppdorf tube for counting cells in Beckman Coulter Type Ac.T5diff http://www.beckmancoulter.com/products/instrument/hematology/act5diffcp.asp.

To stain the platelets with calcein-AM, a 50 ml tube is taken (type polypropylene, Light Protection Tube, www.greinerbioone.com) In this tube, the volume Assaybuffer is added first, needed to dilute the platelets to $2^8$ platelets./ ml . This volume of assay buffer is added first, next the equilvalent volume of Calcein AM is added, to get a final concentration of 2,5 μM Calcein-AM (10 μl Calcein-AM stoack per 10 ml final volume). The tube contents is mixed shortly by vortexing. To this mixture a volume of thrombocytes are added carefully to get a final density of $2^8$ platelets/ml. This mixture is incubated statically for 10-15 min. in the dark to fluorescently stain the platelets.

A new 50 ml tube is taken (type polypropylene, Light Protection Tube, www.greinerbioone.com) to dilute the platelets to $5^7$ cells/ml in assay buffer. After the required volume of assay buffer is added, the equivalent volume of ReoPro stock solution, needed to get a final concentration of 2.5 μg/ml is added, Next the required volume of platelets is added (to obtain $5^7$ platelets/ml). Incubate the tube statically for 10-15 min. in the dark for 10 minutes. Meanwhile rinse the cassette of the Multidrop (Multidrop from Labsystems) thouroughly with assay-buffer.

After the 10 minute incubation with ReoPro, the platelets are further diluted with TBS-Adhäesionsbuffer to $5^5$ platelets/ml (id est 10 000 platelets per 20μl; the volume added to one well) For this final dilution step a polypropylene beaker (volume 500 - 1000 ml) is used. To this beaker the needed volume of TBS-Adhäesionsbuffer is added first, next a sterile floating Stirring Bar is added. The beaker ia put on a magnetic stirring device at setting 3; and the equivalentvolume of platelets is added gently while stirring during 2 minutes to ensure a complete dilution. Next a volume, equivalent to the volume of the cassette of the Multidrop (Multidrop from Labsystems) is thouroughly rinsed with the platelet suspension. Immediately after, the dispersion of thrombos into multiwells is started..The equivalent of the dead volume of tubing and cassette is discarded before. with the Multidrop: 20 μl thrombocyte suspension is added to each well. The system is set up in such a way, that the tubing is as short as possible. Dispension should occur as fast as possible, usually less than 30 minutes are needed to dispense platelets into 30 multiwell plates.

2.6 Incubation & washing

Plates containing platelets and test substances were incubated without lid in the dark at room temperature between 1.5 and 3 h. Next wells were washed with 2 x 60 μl TBS-Washbuffer 1X, paused by 5 minutes while during the plate between the two washes.

Important is, that every corner of the well is washed. This is achieved using an Elx405 96-wells washer (ELx405 Microplate Washer from BioTek Instruments). Aspiration occurred in both the lower left and the upper right corner, dispension always in the same position; -23/0. Next, the plates is turned 180 degrees and the aspiration and dispension is repeated.

2.7 Acumen reading

Plates are read in an Acumen cytometer (Acumen Explorer, TTP Labtech http://www.ttplabtech.com/products/acumen/index.html) using

Software version number 3.1.12 and a MDAQ driver version 1.0.10 (2008-01). Plates were loaded on to the Acumen using the Twister2 (Caliper; www.caliperls.com). The screening room was slightly cooled to appr. 18 - 20 C. Between the unlidded assay plates, there were empty white plates used a lid to avoid condensation of screening plates onto the plate above. The white plates were discarded.

The settings used for reading the plates in the Acumen were described here

Argon-ion laser power set at 6 mW:

Emitted fluorescence was recorded in the following channels using the following settings:

Emitted light with a Wavelength: 500 - 530 nm was recorded in Channel 1:

Data Collection: Enabled, Trigger: Enabled, PMT Voltage: 530 V

Emitted light with a Wavelength: 540 - 560 nm was recorded in Channel 2:

Data Collection: Enabled, Trigger: Enabled, PMT Voltage: 500 V

Emitted light with a Wavelength: 575 - 640 nm was recorded in Channel 3:

Data Collection: Enabled, Trigger: Enabled, PMT Voltage: 500 V

Area of Interest: had a rectangular shape with a width and height of 4 x 4 mm around the center of the well
Acquisition of the emitted light using a thresholding sensitivity of 2 Standard deviations above background values in all recoding channels.
With a Baseline Tracking Window of 301 $\mu$m
And a Noise Smoothing Window of 3 $\mu$m
Object Identification settings were X Overlap: 0.5 $\mu$m, Y Separation: 0.5 $\mu$m
The following polulations were identified using the following filters
Population of platelets (- Filters:

1° Peak Intensity - Baseline: 900 to 20000
1° area: 90 to 1200
Ratio 1° Peak Intensity - Baseline / 3° Peak Intensity - Baseline: 1 to 100

Population of large red material - Filters:

1° Peak Intensity - Baseline: 50 to 200
3° Peak Intensity - Baseline: 80 to 250
3° Perimeter: 45 to 100
3° area: 80 to 300

Population of actived platelets - Filters:

1° Perimeter: 90 to 1000
1° area: 350 to 4000
1° Peak Intensity - Baseline: 333 to 9000
1° Half Width Intensity: 6900 to 1.3e+06
Ratio 1° Peak Intensity - Baseline / 3° Peak Intensity - Baseline: 1 to 50
1° Total Intensity: 65000 to 120000

Population of dim plat (300 1 pibl 1200 - Filters:

1° Peak Intensity - Baseline: 300 to 1200
1° Werea: 90 to 1000
Ratio 1° Peak Intensity - Baseline / 3° Peak Intensity - Baseline: 1 to 100

Population of debris platelets ("debris from platelets") - Filters:

1° Peak Intensity - Baseline: 0 to 20000
1° area: 0 to 90
Ratio 1° Peak Intensity - Baseline / 3° Peak Intensity - Baseline: 0.6 to 100

high ch3 objects - Filters:

1 ° area: 0 to 10000
Ratio 1° Peak Intensity - Baseline / 3° Peak Intensity - Baseline: 0 to 1

From the populations, the following characteristics were extracted:

| | |
|---|---|
| 1. Data: | Number of objects |
| 2. platelets (1 pibl >900): | Number of objects |
| 3. large red material : | Number of objects |
| 4. actived platelets: | Number of objects |
| 5. dim plat (elets): | Number of objects |
| 6. debris platelets: | Number of objects |
| 7. high ch3 objects: | Number of objects |

| | |
|---|---|
| 8. percentage: Number of objects | platelets (1 pibl >900) Number of objects / Data: |
| 9. percentage: >900) Number of objects | actived platelets: Number of objects/ platelets (1 pibl |
| 10. percentage: Number of objects | dim plat (elets): Number of objects/ platelets (1 pibl >900) |
| 11. percentage: platelets: Number of objects | platelets (1 pibl >900) Number of objects / debris |

| | |
|---|---|
| 12. platelets (1 pibl >900): | Median 1 ° Mean Intensity |
| 13. platelets (1pibl >900): | Geometric Mean 1° Mean Intensity |
| 14. platelets (1 pibl >900): | Geometric Mean 1° Peak Intensity - Baseline |
| 15. platelets (1pibl >900): | Geometric Mean 1 ° Total Intensity |
| 16. platelets (1pibl >900): | Mean 1 ° Perimeter |
| 17. platelets (1pibl >900): | Median 1° Werea |

3 Data analysis

3.1 Direct determination of assay quality

For the direct analysis of assay plate quality we calculated a Z' factor as described by Zhanget al.: , "A Simple Statistical Parameter for Use in Evaluation and Validation of High Throughput Screening Assays." J Biomol Screen. 1999;4(2):67-73 , see also http://en.wikipedia.orq/wiki/Z-factor). The Z' factor was calculated using the the parameter: number of platelets (platelets : Number of objects). For the calculation of Z' the data from wells coated with BSA only (a protein with very low binding properties to platelets) were used as low control and data from wells coated with vWF-A1 and subsequently blocked with BSA as high control.

3.2 Multiparameter analysis

Normally, multiple dozen features can be determined for each single treatment. As mostly a small subset of these features is relevant for the discrimination of well-defined control, reference states and possible toxic phenotypes, the most important features were defined for reasons of: data handling, reduction of dimensionality and focused view at the most relevant biological, chemical and pharmacological processes.
The feature selection was obtained by a thorough design and construction of appropriate classifier (such as K-nearest neighbour (knn) & Naive Bayes) They were validated by cross-validation (p = ¾) and fifty re-sampling iterations. As a result, these classifiers could distinguish quite accurately the well characterised control -, reference - and possible toxic phenotypes. In order to identify the most meaningful features of the classifier, an importance analysis was conducted. These results have been compared with the results of a correlation analysis. This approach did allow the selection of the most important and least correlated feature set. The resulting feature set was used during HTS and listed above.

3.3 Equipment used

| | |
|---|---|
| Acumen Explorer | TTP Labtech |

http://www.ttplabtech.com/products/acumen/index.html

Softwwere version 3.1.12 , MDAQ driver version 1.0.10 (2008-01).

| | |
|---|---|
| Biomek FX | Beckman Coulter |
| Multidrop | Thermo Labsystems |
| Vi-Cell™ XR | Beckman Coulter |
| Minifuge T | Heraeus |
| ELx 405 Select | Biotek |
| Twister2 | Caliper |

4. Results

4.1 Labelling platelets with Calcein-AM

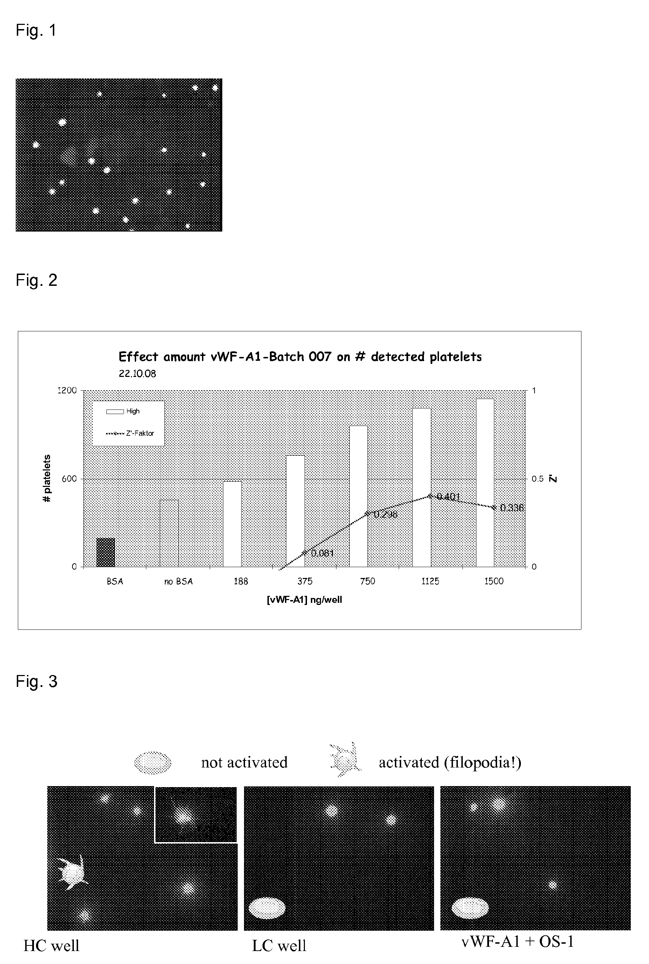

To detect the platelets after adhesion to the vWF-a1 coated wells, the washed-platelets were fluorescently labeled by calcein-AM. Microscopical analysis using filters suitable to detect calcein-AM showed bright spherical objects with no clear morphological signs of preactivation. However, a clear vage fluorescent background staining was also frequently observable as well as larger, bright structures, often not in focus and probably representing labelled blood cells, see Fig. 1.

4.2 Adhesion of labeled plates to wells coated with vWF-A1

Fluorescently-labelled washed platelets were allowed to adhere to immobilized vWF-A1. Adherent platelets were quantitated via imaging on the Acumen using the settings described above. Several amounts of vWF-A1 were added to the plates and the amount of attached platelets was determined in 96-wells format after washing. The data in Fig. 2 shows that the amount of detected platelets increased with increasing amounts of vWF-A1. However, also in untreated wells high amounts of platelets could be observed. This is caused by the attachment of platelets to the plastic wells, and this attachment of platelets to (uncoated) wells could be greatly reduced by coating the wells with BSA. To determine the optimal amount of vWF-A1 needed for this assay, Z' values were calculated. From the data it was clear that a further increase in the amount of A1 above 1 $\mu$g/ well does not improve the assay and that the minimal requirements of a Z' value > 0.5 (see http://en.wikipedia.orq/wiki/Z-factor) was not yet attained, see Fig. 2.

4.2 Activation of platelets after dispersion

The dispersion of the platelets might induce a partial activation. Activated platelets have different adhesive properties compared to resting platelets. During activation some proteins were actively translocated from the interior of the platelet to the plasma membrane. Also the clearance of receptors from the surface has been described by Hourdill et al.; von Willebrand factor bound to glycoprotein Ib Is cleared from the platelet surface after platelet activation by thrombin. In: Blood : 79, pp. 2011-2021, 04/15/1992.

To check if the dispension of platelets caused any activation in sense of any change in morphology, the platelets were observed by high-resolution fluorescence microscopy microscope immediately after dispension. Platelets interacting with A1 had several extensions, indicating that activation had occurred. But no morphological signs of activation of the platelets is observed in wells were plates should not get activated (BSA or well with sufficient inhibitor), indicating that the procedure of labeling and dispensing platelets did not induce any activation as observed by changes in morphology, see Fig. 3.

4.4 Optimisation of wash programs

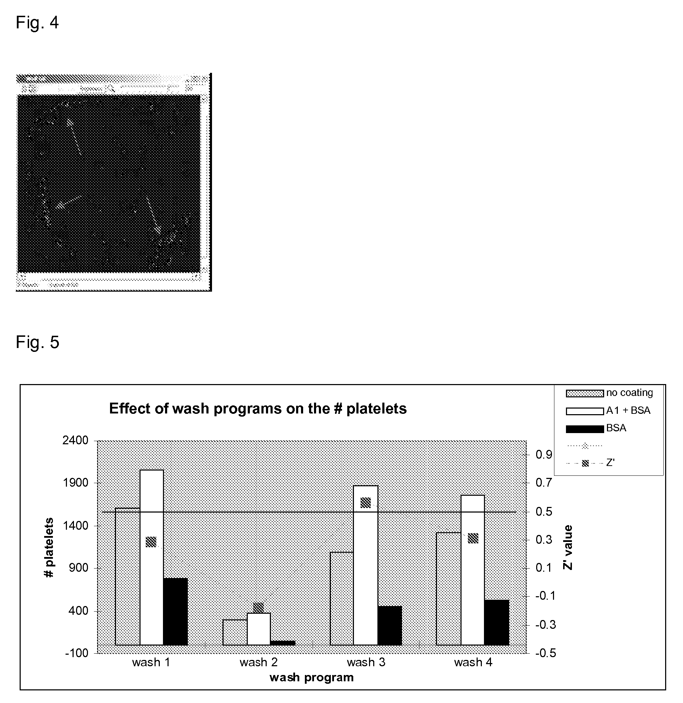

When observing the wells it became clear that the wash setting could be optimized. In many low control wells significant amounts of platelets remained attached in the well, see figure 4. The attached platelets showed irregular patterns that caused by inefficient washing of certain wereas of the well.

To improve the assay, many variations of washer settings (wash programs) were tested. It became clear, that a careful testing of washer programs could improve the statistics of the assays. Figure 5 shows the effect of several washer programs on the Z' value of the assay.

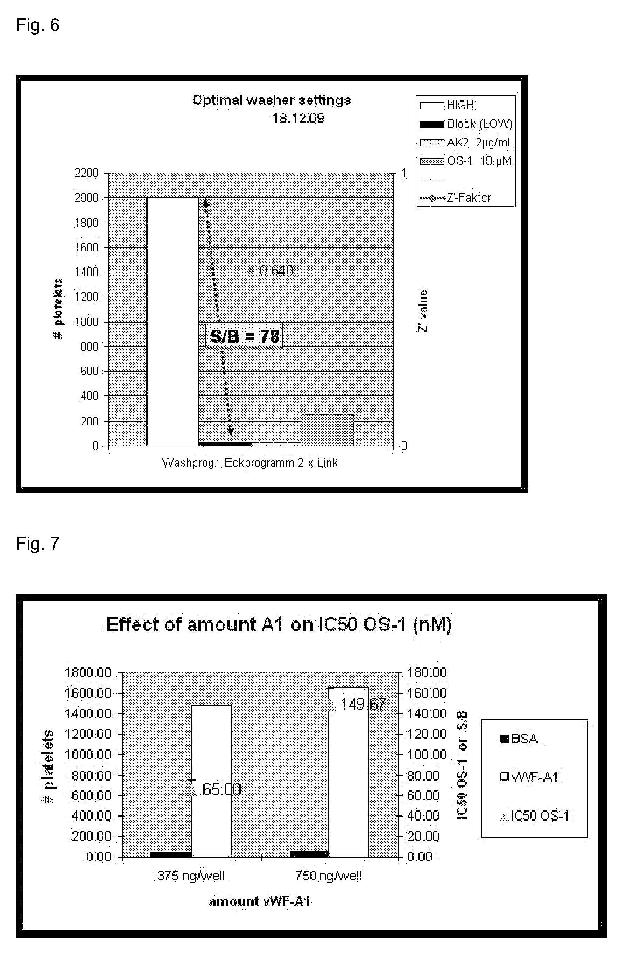

By analysing the images generated from the wells by whole well cytometry and by comparing the statistics of the plate, the washers settings could be optimized to a S/B from 78 and a Z' of > 0.6. using these setting, an almost 90% inhibition of platelets adhesion was achieved when an antibody is directed against the platelet glycoprotein gplb alpha (AK2; see section 1.7) and OS-1 were used as inhibitors at 2$\mu$g/ml and 10 $\mu$M, respectively, see Fig. 6.

4.5 Determining the sensitivity of the assay via the IC50 value for the specific inhibitor OS-1

The specific inhibitor OS-1 was discovered in 2008 by Bernard et al.. Biochemistry 2008, 47, 4674-4682. Using ELISA technology an IC50 value was of 510 nM was determined for the ability of OS-1 to inhibit the interaction between vWF-A1 and GPIb interaction. To determine if the developed assay could detect the inhibiting ability of OS-1 with at least similar sensitivity, the IC50 values for OS-1 was determined.
It was observed that the IC50 values were dependent on the amount of A1 used for coating the well of a 384 plate. Therefore, the amounts of A1 were minimized to optimally use the limited amount of isolated A1 protein, but still getting sufficient statistical values for a valid assay. Using the optimized wash programs, lower amounts of A1 could be used. With these lower amounts of vWF-A1 IC50 for OS-1 were obtained varying ca 65 and 150 nM, dependent on the amount of A1 used for coating. These IC50 were closed to the 510 nM value published by Benard et al. , This indicates that the described assay is at least as sensitive than the assay described by Berard et al 2008, see Fig. 7.

4.6 Stability of plates during screening & determination of the throughput of the assay

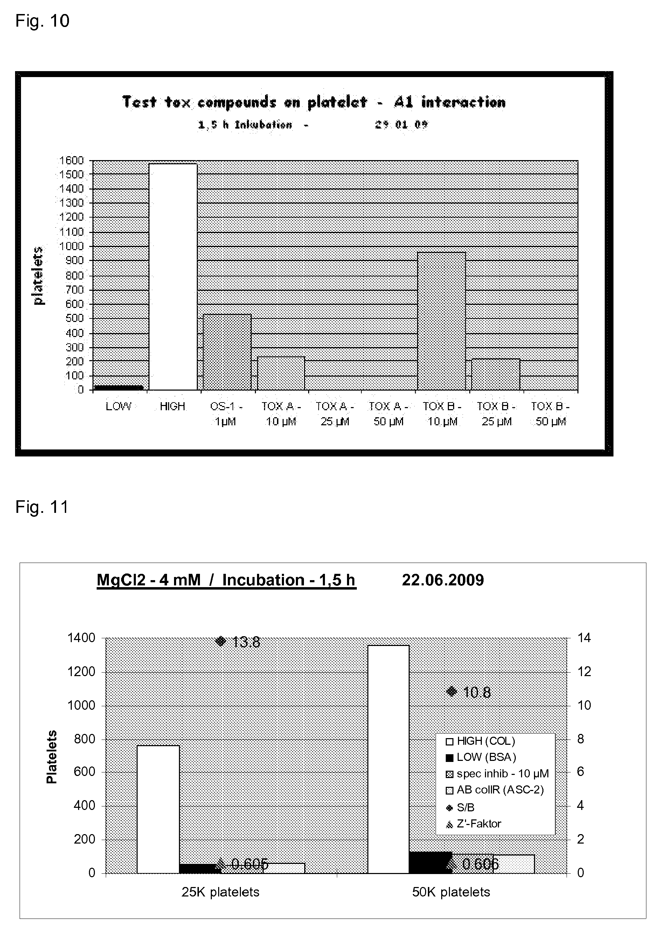

After the methods to get to a meaningful assay had been set, the stability of the plates was determined. This was necessary since during the assay an accumulation of plates occurs because the washing of the plates (ca 5 min /plate) and reading of the plates (10 min/plate) is slower than the dispensing step (1 min/plate). This means that the plates that were dispensed last need to be stored upto 9 hours before being read. Therefore t it was determined if the plates could be stored (at room temperature) before washing, meaning a prolonged test substance incubation time and if the plates could be stored after washing; before reading the plates.

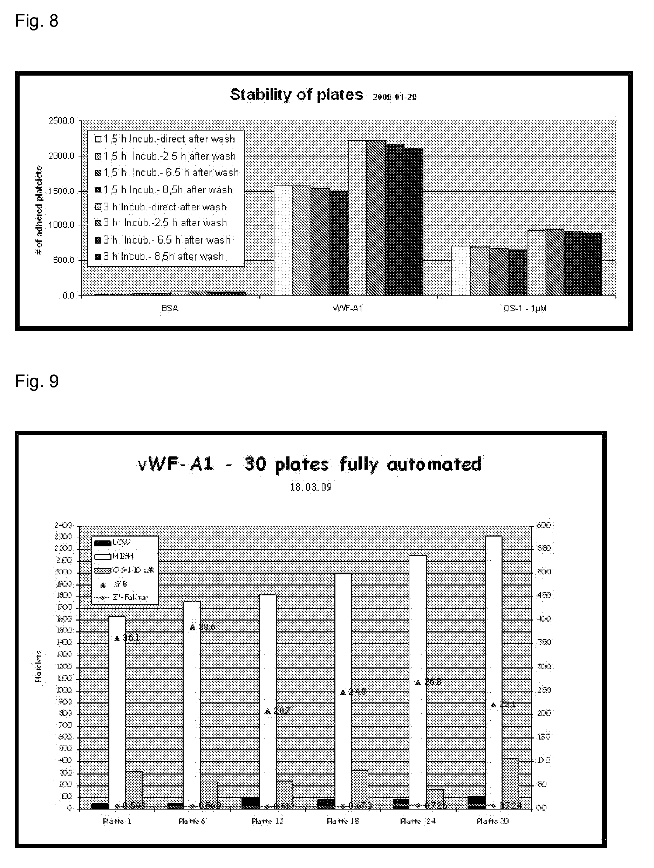

Figure 8 shows the effect of plate store on the assay. An increase in plate storage time increased the number of detected platelets. Apparently more platelets were able to settle and adhere. The prolonged storage before washing resulted in a slight loss of the number of detected platelets, but this did not have an effect on the statistical significance of the assay. In all cases the Z' was > 0.5.
Further it was observed, that the toxic effects of test substances with known toxicity issues (see section below) increased with increasing storage times (data not shown). In contrast, the reference inhibitor OS-1 showed almost constant values, see Fig. 8.
Knowing the time that the plates could be stored before reading, a set of 30 plates was tested under automated conditions. The data of this test is presented in the figure below, where the results of the first, 6th, 12th, 18th, 24th and the last plate were shown. In all plates of this run, a S/B of at least 20x was obtained with a Z' value of > 0.5 and a high inhibition by OS-1. It was therefore concluded that the assay is suitable for high-throughput screening with at least 30 plates per day, see Fig. 9.
The assay identifies toxic test substances as inhibitors when the # of adhered platelets is used a single parameter for analysis. In many cellular assays aimed to identify inhibitors, test substances with toxic features were identified as inhibitors. Therefore two test substances were tested, known for the toxic effects in many cell-based assays.
Both toxic test substances showed substantial inhibition at 10 $\mu$M with a complete inhibition of platelet adhesion to vWF-A1 at 30 $\mu$M concentration. See Fig. 10.
We therefore concluded that the assay will identify toxic test substances as inhibitors when only the number of attached platelets is used as a read out. To avoid an accumulation of false positives during screening, it was attempted to reduce the number of false positives by the identification of toxic test substances.

4.8 Discrimination of "toxic test substances" from inhibitors

To reduce the number of false positives due to toxicity of the test substances, two strategies were used:

Either counter-screening (testing all the inhibitors in an assay where adhesion onto collagen is measured) or multi-parameter analysis.

4.8.1 Identification of toxic test substances via (counter) screening on interaction with collagen1: practical description of the assay

For this assay essentially the same set up was used as described above for testing the adhesion of platelets onto A1-coated plates.
To coat wells with rat tail collagen1, a stock solution consisting of 200 $\mu$g/ml Collagen I (rat tail, SIGMA (C8897)) in 0,01 M acetic acid was prepared. This solution was diluted with 0,01 M acetic acid to 10 $\mu$g/ml and 25 $\mu$l was added to a 384 well. Plates were incubated overnight at 4C for binding of collagen1 to the well. One hour before test substance addition, the wells of the plates were blocked with BSA as described above. Also the preparation, labeling and dispension of platelets is as described for vWF-A1. For the platelet adhesion to collagenl, the concentration of MgCl2 was increased to 4 mM and the number of platelets added per well to 50 000 platelets. Using these conditions, the assay was able to detect the interaction of specific inhibitors with good statistical parameters, see Fig. 11.

A set of 6 plates were tested under automated conditions. For all plates a Z' value above 0.5 was obtained with a S/B of 20 or more.

A training set composed 69 test substances in total was used to validate this assay. From these test substances, 31 test substances were known to be putative toxic test substances, 2 putative inhibitors of vWF-A1 -platelet interaction, 32 collagen-platelet interaction inhibitors and 4 test substances, known to be toxic in many cellular assays, were tested at 10 $\mu$M in the platelet - collagen interaction assay.

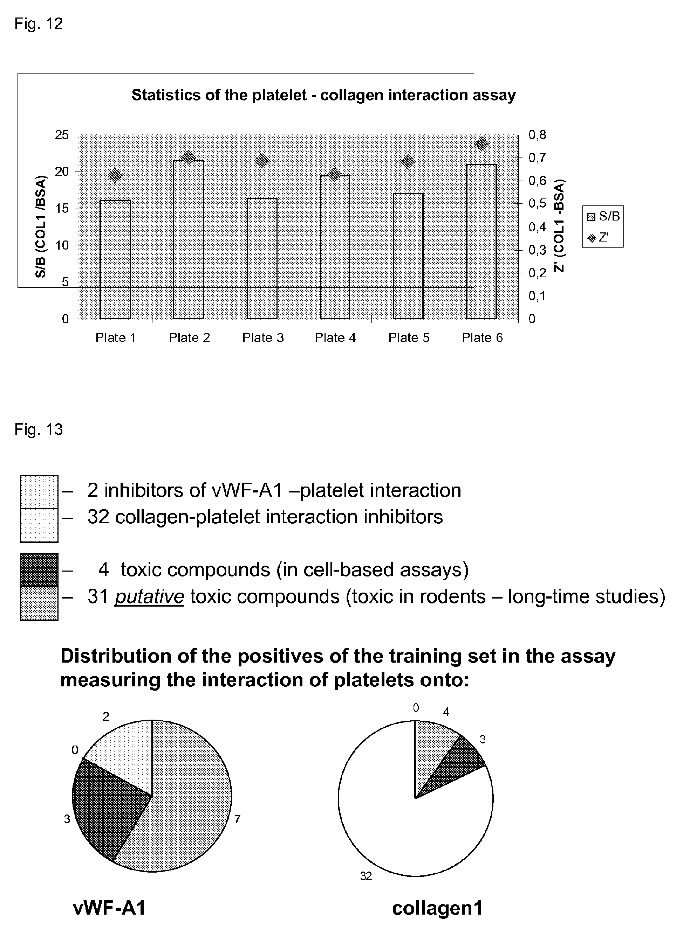

A summary of the data were shown in Fig. 13:

More than 75% of the test substances that acted as inhibitors in the vWF-A1 assay, came from a group of test substances that were classified as potentially toxic. From the 4 test substances known to be toxic in most cellular assays, 3 were toxic. The same test substances were toxic in the platelet - collagen interaction assay. Also all 32 known collagen inhibitors could be indentified, with the same ranking (based on IC50s, data not shown). On the other hand, bona-fide inhibitors from the platelet - A1 interaction, like the reference test substance OS-1 and the antibody against GP1 b (AK2) did not inhibit the interaction with collagen. Therefore it was concluded that the assay for interaction between platelets and collagen 1 is able to detect collagen inhibitors and when used as a counterscreen is able to detect most test substances with cytotoxic effects.

4.8.2 Detected platelets look different after incubation with toxic test substances

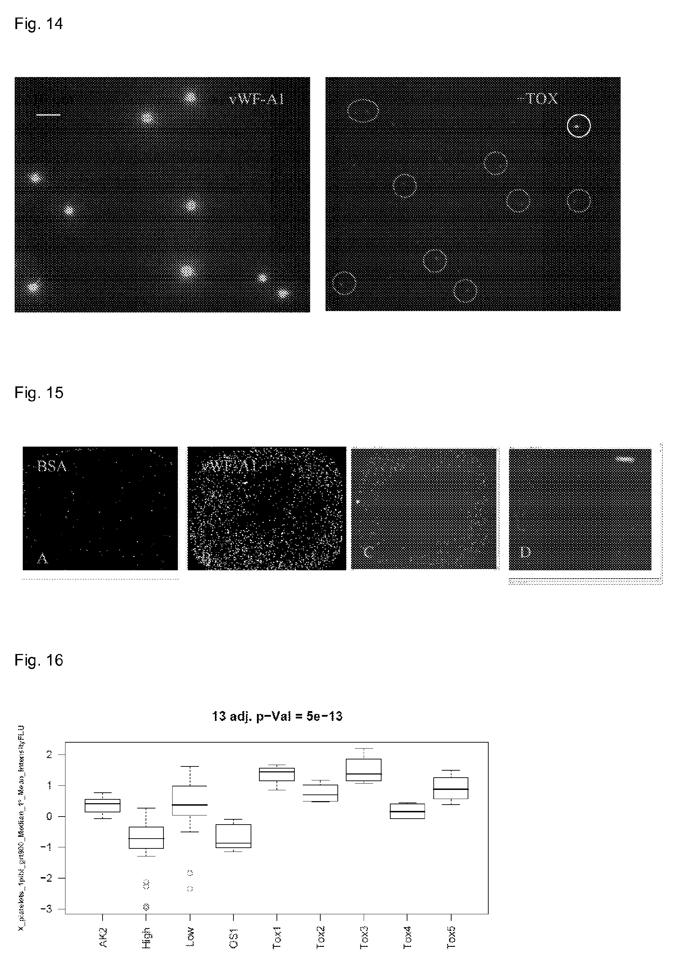

By looking at the wells by microscopy, small punctate fluorescent material was found instead of the platelets, see Fig. 14. We interpreted this material as remnants from platelets that were removed after washing and therefore called it "debris". However it can not be excluded that this small material represents other material like vesicles. Using other putative toxic test substances, oversized platelets or platelets with reduced fluorescence intensity were observed with reduced fluorescence intensity.

In the Acumen in wells where platelets were incubated with toxic test substances also a lot of small punctuate fluorescent material was observed. In these wells the amount of platelets was reduced and often the remaining platelets were less fluorescent. See Fig. 15.

Therefore we tried to discriminate between a toxic test substance and a non toxic inhibitor by using multi-parameter analysis.

4.9 Multi-parameter analysis: setup

Feature selection

The aim of the assay is to identify test substances which inhibit the vWF-A1 platelet interaction. However the perfect inhibitor of the vWF-A1 platelet interaction could also be regarded as a toxic test substance for platelets. Therefore following control and reference states (stimulator (low)-, neutral (high) control, AK2, OS1, toxic group 1 to 5) were considered and selected as important groups.

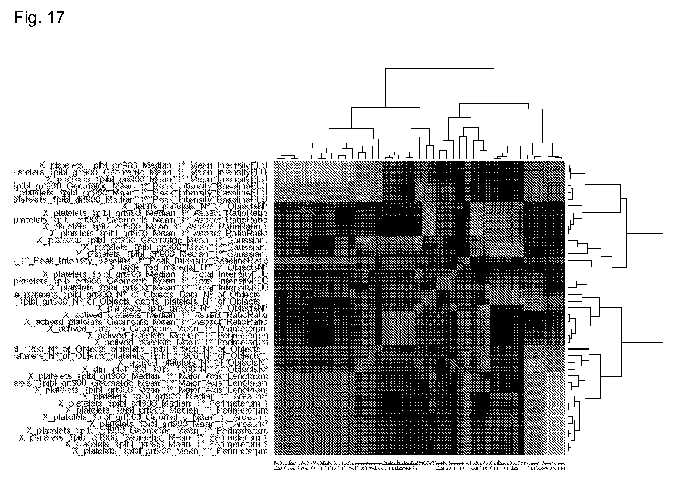

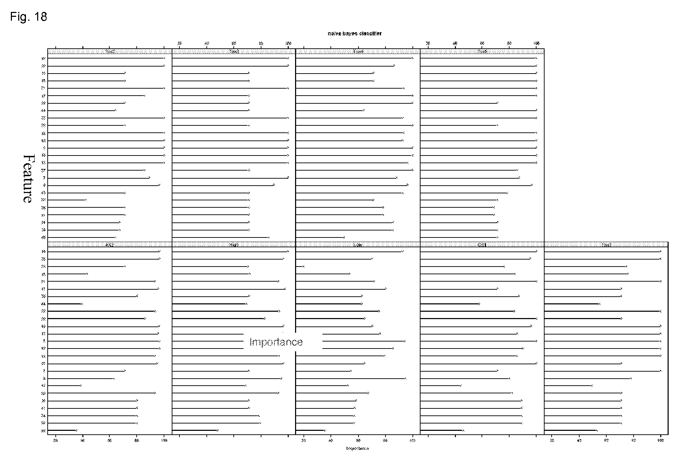

To identify the most meaningful features for the distinction of the selected groups, it is necessary to use in a first step all possible parameters describing the fluorescent intensity or morphology of individual platelets, small groups of platelets or objects that might be derived from platelets or other blood cells. In a first step, each single feature was tested in respect of the statistical significance to be able to distinguish at least one of the nine important groups (Fig.16). Secondly a correlation analysis was performed (Fig. 17). It is very easy to identify that within the set of 43 features there were sets of highly correlated ones. As a third selection criteria the assessment of all features in respect of the importance of a constructed classifier (k-nearest neighbour (knn) and Naive Bayes (cross validation (p = ¾), fifty re-sampling iterations)) was taken into account (Fig. 18).

The following set of features has been identified. They represent these ones out of the 43 possible features, which were least correlated between each other and were of importance for the constructed classifier:

platelets (1pibl >900): Median 1° Mean Intensity (FLU)
actived platelets: N° of Objects (N°)
platelets (1pibl >900): Geometric Mean 1 ° Peak Intensity - Baseline (FLU)
platelets (1pibl >900): Geometric Mean 1 ° Total Intensity (FLU
platelets (1pibl >900): Median 1 ° Werea ($\mu$m$^2$)
platelets (1pibl >900): Mean 1 ° Perimeter ($\mu$m)
debris platelets: N° of Objects (N°)

Percentage dim platelets (300 1pibl 1200: N° of Objects / platelets (1pibl >900): N° of Objects) (%)

platelets (1pibl >900): Geometric Mean 1° Mean Intensity (FLU)

| | |
|---|---|
| percentage: objects | platelets (1 pibl >900) Number of objects / Data: Number of |
| percentage: Number of objects | actived platelets: Number of objects/ platelets (1 pibl >900) |
| percentage: Number of objects | dim platelets: Number of objects/ platelets (1 pibl >900) |
| percentage: Number of objects | platelets (1 pibl >900) Number of objects / debris platelets: |
| (as described in the section: Acumen reading.) | |

4.9.2 Classifier assessment

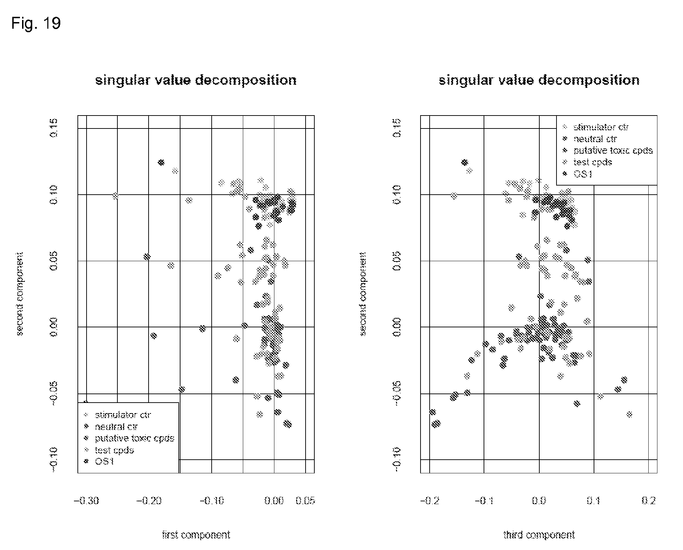

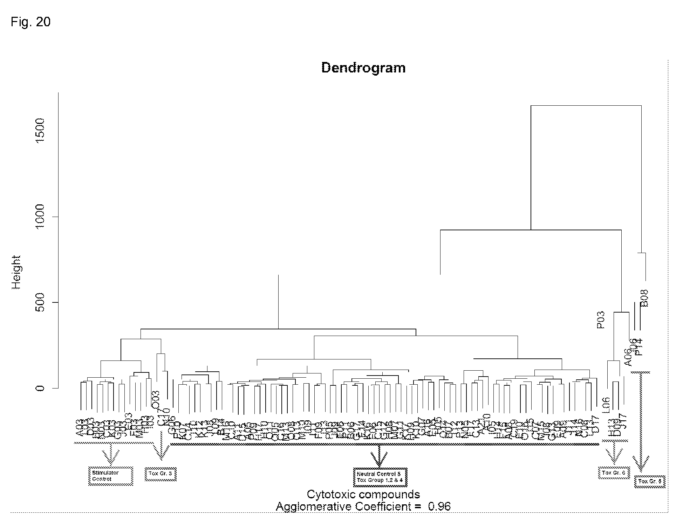

Before the potential hits of the main screen were being detected, the constructed classifier has to be evaluated. Therefore test data was obtained in HTS mode under automated conditions. The test data comprised as small set of selected test substances such as known inhibitor, known toxic test substances and neutral - and stimulator controls. Singular value decomposition was first applied to this data set (Figure 19). Although the dominant factor hidden behind the first and third component is not quite clear, the second component accounts mainly for the distinction of the stimulator (green) - and neutral control (red). The positive reference OS1 is nicely scattered within the area of the stimulator control. This result is quite encouraging for the construction of a classifier. According to the results of the singular value decomposition the following groups - classes have been identified: stimulator -, neutral control, OS1 (reference) and potential toxic test substances. The potential toxic test substance group is very diverse. Therefore this group was thoroughly investigated. Based on the hierarchical cluster analysis of the putative toxic test substances with the stimulator - and neutral controls, three interesting potential toxic groups have been identified for the construction of a classifier (accuracy - 0.91, 95% confidence interval - (0.836, 0.958) and kappa - 0.8866) (Fig. 20).

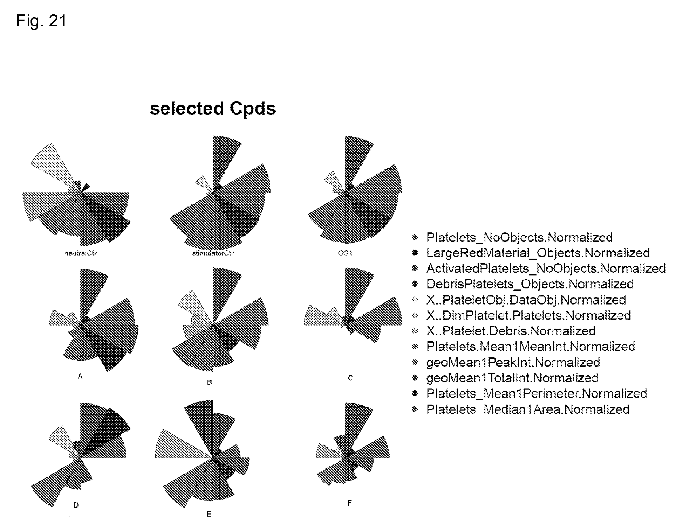

A small subset set of selected test substances were shown in Figure 21. Due to the 12 dimensionality of the whole data vector for each measured test substance, a so called star plot representation is chosen. This type representation allows quickly visual assessment of group assignments. The selected tool test substances were more similar to the OS1 reference - and stimulator control state than to the potential toxic groups and the neutral control state.

These few examples illustrate further, that it is expected that the multi-parameter analysis is able to reduce the number of false positive test substances by discriminating between toxic test substances and more specific non-toxic inhibitors.

**Claims**

1. A method for detecting and/or quantifying the interaction of platelets or a platelet surface molecules with an interaction partner comprising the steps of

    (a) providing a multiwell plate amenable for optic analysis by laser scanning or fluorescence cytometry or microscopy, wherein one or more wells of the plate are coated with an interaction partner of a platelet surface molecule

    (b) bringing into contact of labeled platelets with

        i) one or more interaction partner -coated wells and one or more of ii, iii or iv:

        ii) one or more wells (of the above or an additional multiwell plate) coated with one or more molecules known not to interact specifically or strongly with platelets or platelet surface molecules (low control)

        iii) one or more wells (of the above or an additional multiwell plate) coated with one or more molecules known to specifically interact with platelets or platelet surface molecules (high control)

        iv) one or more wells (of the above or an additional multiwell plate) coated with one or more molecules known to bind but not to activate platelets (activation control) III and Iv can be the same

    (c) washing the wells to remove unbound platelets

    (d) detecting by laser scanning or fluorescence microscopy or cytometry in a pre-selected area of the wells or of the plate

        i) the total number of platelets, and/or

ii) the number of activated platelets,

wherein interaction partners with strong platelet interaction can be identified based on a high number of detected platelets in comparison to the low control and or a high number of activated platelets in comparison to the low control, whereas partners with weak or low platelet interaction can be identified based on a low number of detected platelets in comparison with the high control and/or a low number of activated platelets in comparison to the high control.

2. Method according to claim 1, wherein one of the following parameters is determined:

a) the number of platelets with weakened fluorescence signal
b) the number of platelets with aberrant morphology (fragments)
c) the number of objects with aberrant, red-shifted fluorescence (large red material), and
wherein a high number in either of a, b or c detected for b i) of claim 1 in comparison to b iii or iv) of claim 1 (the high or activation control) acts as an identifier for toxic or aberrant effects of the interaction partner.

3. A method for detecting and/or quantifying the capability of a test substance to modulate the binding of a platelet or platelet surface molecule with an interaction partner comprising the steps of:

(a) providing a multiwell plate amenable for optic analysis by laser scanning or fluorescence cytometry or microscopy, wherein one or more wells of the plate are coated with an interaction partner of a platelet surface molecule
(b) bringing into contact of labeled platelets with

i) one or more interaction partner -coated wells and optionally with one or more of ii, iii or iv:
ii) one or more wells (of the above or an additional multiwell plate) coated with one or more molecules known not to interact specifically or strongly with platelets or platelet surface molecules (low control)
iii) one or more wells (of the above or an additional multiwell plate) coated with one or more molecules known to specifically interact with platelets or platelet surface molecules known not to specifically or strongly interact with and/or activate platelets (high control)
iv) one or more wells (of the above or an additional multiwell plate) coated with one or more molecules known to bind but not to activate platelets (activation control) III and Iv can be the same

(c) bringing into contact of a test substance with the platelets
(d) washing the wells to remove unbound platelets
(e) detecting by laser scanning or fluorescence microscopy or cytometry in a pre-selected area of the wells or the plate the total number of platelets and optionally
(f) detecting by laser scanning or fluorescence microscopy or cytometry in a pre-selected area of the wells one or more of the following parameters I, ii, iii or iv:

i) the number of platelets with weakened fluorescence signal
ii) the number of platelets with aberrant morphology (fragments)
iii) the number of objects with aberrant, red-shifted fluorescence (large red material),
iv) the number of activated platelets, and

(g) comparing the values according to (e) and optionally (f) with one or more reference values generated without addition of test substance,
wherein a difference between the values detected with and without test substance indicates that the test substance is capable of modulating the binding of the platelet surface molecule or of platelets.

4. Method according to claim 3, wherein one or more of f) i, ii or iii or is detected and wherein an increase between one or more of the values of f) i, ii, iii or iv) in comparison to a reference sample without test substance is indicative of toxic effects of the test substance.

5. Method according to claim 3 or 4, wherein the number of activated platelets according to f) iv is detected and wherein an increase between in comparison to a reference sample without test substance is indicative platelet-activating effects of the test substance.

6. A method according to one of claims 2 to 5, wherein the platelets are mixed with the test substance prior to bringing into contact of test substance and platelets with the coated plate.

7. A method according to one of the previous claims wherein the platelets are labelled with a fluorescent dye such as one of the following: calcein-AM, CMFDA (5-chloromethylfluorescein diacetate), PKH67, resazurin, Lavacell, (fixable) FM1-43, Nano-Orange or fluorescently-labelled phalloidin....label/dye: so definieren, daß er ein detektierbares Signal im micro/cyto gibt

8. The method according to one of the previous claims, wherein the platelet surface molecule is a platelet surface protein, such as: GP1 b, the collagen receptor, PAR1, PAR4, P2Y purinoceptor 1, P2Y purinoceptor 12, junctional adhesion molecule A (JAMA), JAMC

9. The method according to one of the previous claims, wherein the interaction partner of the platelet surface receptor is a naturally occurring interaction partner, a functional derivative or fragment thereof or a synthetic interaction partner.

10. The method according to claim 9, wherein the interaction partner is one of the following: the van Willebrand factor 1 (vWF-1), the A1 domain of vWF-1, collagen, fibrinogen, Protein c, Mac-1 ($\alpha$M$\beta$2), P-selectin, (CD62P), High-molecular weight kininogen, Thrombospondin-1, soluble GPVI), PSGL1, or a functional fragment or derivative of any of these.

11. The method according to one of the previous claims, wherein the multiwell plate is a 96-well, a 386-well or a 1536-well plate and preferably a 386-well plate.

12. Article of manufacture comprising a leaflet with instructions for the method according to one of the previous claims.

Fig. 1

Fig. 2

**Effect amount vWF-A1-Batch 007 on # detected platelets**
22.10.08

Fig. 3

not activated     activated (filopodia!)

HC well        LC well        vWF-A1 + OS-1

Fig. 4

Fig. 5

Effect of wash programs on the # platelets

Fig. 6

Fig. 7

Fig. 8

Fig. 9

Fig. 10

Test tox compounds on platelet - A1 interaction

1,5 h Inkubation - 29.01.09

Fig. 11

MgCl2 - 4 mM / Incubation - 1,5 h    22.06.2009

□ HIGH (COL)
■ LOW (BSA)
⊠ spec inhib - 10 µM
□ AB collR (ASC-2)
◆ S/B
▲ Z'-Faktor

Fig. 12

**Statistics of the platelet - collagen interaction assay**

Fig. 13

- 2 inhibitors of vWF-A1 –platelet interaction
- 32 collagen-platelet interaction inhibitors

- 4 toxic compounds (in cell-based assays)
- 31 *putative* toxic compounds (toxic in rodents – long-time studies)

**Distribution of the positives of the training set in the assay measuring the interaction of platelets onto:**

vWF-A1

collagen1

Fig. 14

Fig. 15

Fig. 16

Fig. 17

Fig. 18

EP 2 431 739 A1

Fig. 19

singular value decomposition

singular value decomposition

Fig. 20

Dendrogram

Cytotoxic compounds
Agglomerative Coefficient = 0.96

Fig. 21

**selected Cpds**

* Platelets_NoObjects.Normalized
* LargeRedMaterial_Objects.Normalized
* ActivatedPlatelets_NoObjects.Normalized
* DebrisPlatelets_Objects.Normalized
* X..PlateletObj.DataObj.Normalized
* X..DimPlatelet.Platelets.Normalized
* X..Platelet.Debris.Normalized
* Platelets.Mean1MeanInt.Normalized
* geoMean1PeakInt.Normalized
* geoMean1TotalInt.Normalized
* Platelets_Mean1Perimeter.Normalized
* Platelets_Median1Area.Normalized

Europäisches
Patentamt

European
Patent Office

Office européen
des brevets

**EUROPEAN SEARCH REPORT**

Application Number

EP 10 30 6008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RATHORE V ET AL: "Phospholipase Cgamma2 contributes to stable thrombus formation on VWF", 27 August 2004 (2004-08-27), FEBS LETTERS, ELSEVIER, AMSTERDAM, NL, PAGE(S) 26 - 30, XP004536196, ISSN: 0014-5793 * figure 1 * | 1-4, 6-10,12 | INV. G01N33/49 G01N33/86 |
| X | ARTONI ANDREA ET AL: "ADAMTS-13 Binds Platelets through Gpib", 1 November 2009 (2009-11-01), BLOOD, AMERICAN SOCIETY OF HEMATOLOGY, US, PAGE(S) 1186/3044, XP009147500, ISSN: 0006-4971 * abstract * | 1-6,8-12 | |
| X | YA A NAIMUSHIN ET AL: "Ability of Different Glycoprotein IIb-IIIa Ligands to Support Platelet Aggregation Induced by Activating Antibody CRC54", BIOCHEMISTRY (MOSCOW), KLUWER ACADEMIC PUBLISHERS-PLENUM PUBLISHERS, NE, vol. 70, no. 7, 1 July 2005 (2005-07-01), pages 782-789, XP019294640, ISSN: 1608-3040 * page 784, column 1 * | 1,9,10, 12 | TECHNICAL FIELDS SEARCHED (IPC) G01N C12N C07K |
| A | WO 2010/040861 A1 (UNIV DUBLIN CITY [IE]; ROYAL COLLEGE OF SURGEONS IN I [IE]; BASABE-DES) 15 April 2010 (2010-04-15) * figures 7,9; examples 3,5 * | 1-12 | |

-/--

The present search report has been drawn up for all claims

| Place of search | Date of completion of the search | Examiner |
|---|---|---|
| Munich | 12 May 2011 | Mason, William |

EPO FORM 1503 03.82 (P04C01)

Europäisches
Patentamt

European
Patent Office

Office européen
des brevets

## EUROPEAN SEARCH REPORT

Application Number

EP 10 30 6008

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHEN DONG ET AL: "A new plate based plasma von Willebrand factor (VWF): Ristocetin cofactor activity assay", BLOOD, AMERICAN SOCIETY OF HEMATOLOGY, US, vol. 110, no. 11, part 1, 1 November 2007 (2007-11-01), page 638A, XP009147521, ISSN: 0006-4971 * abstract * | 1-12 | |

-----

TECHNICAL FIELDS
SEARCHED       (IPC)

The present search report has been drawn up for all claims

| Place of search | Date of completion of the search | Examiner |
|---|---|---|
| Munich | 12 May 2011 | Mason, William |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

..............................................................................................

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 10 30 6008

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2011

| Patent document cited in search report | Publication date | Patent family member(s) | Publication date |
|---|---|---|---|
| WO 2010040861 A1 | 15-04-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- database. NP_000543.2
- **TITANI K ; KUMARS ; TAKIO K ; ERICSSON LH ; WADE RD ; ASHIDA K ; WALSH KA ; CHOPEK MW ; SADLER JE ; FUJIKAWA K.** Amino acid sequence of human von Willebrand factor. *Biochemistry,* 03 June 1986, vol. 25 (11), 3171-84
- **BENARD, S.A. ; SMITH, T.M. ; CUNNINGHAM, K. ; JACOB, J. ; DESILVA, T. ; LIN, L. ; SHAW, G.D. ; KRIZ, R. ; KELLEHER, K.S.** *Biochemistry,* 2008, vol. 47 (16), 4674
- **KAGEYAMA, S. ; YAMAMOTO, H. ; NAKAZAWA, H. ; YOSHIMOTO, R.** *Thromb. Res.,* 2001, vol. 101, 395-404
- **BLUE.** Application of high-throughput screening to identify a novel αIIb-specific small-molecule inhibitor of αIIb3-mediated platelet interaction with fibrinogen. *Blood,* 01 February 2008, vol. 111 (3), 1248-1256

- **SHRIMPTON.** *Journal of Experimental Medicine,* vol. 196 (8), 1057-1066
- *Drugs of the Future,* 2003, vol. 28 (1), 61-67
- **BENARD et al.** *Biochemistry,* 2008, vol. 47 (16), 4674-4682
- **ZHANG et al.** A Simple Statistical Parameter for Use in Evaluation and Validation of High Throughput Screening Assays. *J Biomol Screen,* 1999, vol. 4 (2), 67-73
- **HOURDILL et al.** von Willebrand factor bound to glycoprotein Ib Is cleared from the platelet surface after platelet activation by thrombin. *Blood,* 15 April 1992, vol. 79, 2011-2021
- **BERNARD et al.** *Biochemistry,* 2008, vol. 47, 4674-4682